# EUROPEAN PATENT APPLICATION

(11) **EP 4 066 726 A1**
(43) Date of publication of application: **05.10.2022**
(21) Application number: 22163725.9
(22) Date of filing: 23.03.2022
(51) Int. Cl.: A61B 1/05, A61B 1/00, G02B 23/24

(54) **COMPOUND-EYE ENDOSCOPE**

(30) Priority: 30.03.2021 JP 2021057680
(71) Applicant: Panasonic i-PRO Sensing Solutions Co., Ltd., Fukuoka-shi, Fukuoka 812-8531 (JP)
(72) Inventor: KOHNO, Haruhiko, Fukuoka, 812-8531 (JP); SHIRAI, Naomi, Fukuoka, 812-8531 (JP)
(74) Representative: Eisenführ Speiser

(57) **Abstract**

A compound-eye endoscope includes two or more image-capturing modules, a sub-frame, and an outer shell. The two or more image-capturing modules are formed in the same outer shape, and each of the image-capturing modules includes a lens barrel housing an optical system, an image sensor, and a sensor holding member that relatively fixes the lens barrel and the image sensor. The sub-frame relatively fixes each of the two or more image-capturing modules. The outer shell accommodates and fixes the sub-frame and the two or more image-capturing modules.

## Description

### TECHNICAL FIELD

The present disclosure relates to a compound-eye endoscope.

### BACKGROUND ART

In the related art, there has been known an electronic endoscope apparatus for medical use or industrial use that is highly reliable and is capable of obtaining a general image and a high-quality stereoscopic image (see, for example, Patent Literature 1). A video scope of the electronic endoscope apparatus has a double hermetic sealing structure including: an internal structure that is provided with an image-capturing unit at a tip end portion thereof; and an exterior structure that serves as an exterior of the internal structure. The exterior structure has a configuration in which an outer cylinder having a cover glass at a tip end portion thereof, and an operation portion provided at a rear end portion of the outer cylinder and having a video signal extraction unit are hermetically sealed. Inside the exterior structure, the internal structure is provided in which an inner cylinder provided with an image-capturing unit having an objective lens and a CCD module at a tip end portion thereof and a video signal connection portion provided at a rear end portion of the inner cylinder are hermetically sealed.

### CITATION LIST

### PATENT LITERATURE

Patent Literature 1: JP-H09-265047-A

A 3D endoscope capable of obtaining a captured image having left and right parallax and displaying the captured image stereoscopically is configured by assembling two image sensors and two lens barrels (optical systems) to one component. However, in the electronic endoscope apparatus disclosed in Patent Literature 1, a CCD module in which a CCD module for a left eye and a CCD module for a right eye are integrated is inserted into a hollow portion of a mounting member formed in a box shape, and is fixed by a screw, an adhesive, or the like. Therefore, there is a problem that the structure of the mounting member becomes complicated, which is not suitable for mass production, and the manufacturing cost of the 3D endoscope increases.

### SUMMARY OF INVENTION

The present disclosure has been made in view of the above-described circumstances in the related art, and an object thereof is to provide a compound-eye endoscope that is excellent in mass productivity and capable of reducing the manufacturing cost.

The present disclosure provides a compound-eye endoscope including: two or more image-capturing modules each of which includes a lens barrel housing an optical system, an image sensor, and a sensor holding member that relatively fixes the lens barrel and the image sensor; a sub-frame that relatively fixes each of the two or more image-capturing modules; and an outer shell that accommodates and fixes the sub-frame and the two or more image-capturing modules.

According to the present disclosure, it is possible to provide a compound-eye endoscope excellent in mass productivity and capable of reducing the manufacturing cost.

### BRIEF DESCRIPTION OF DRAWINGS

Fig. 1 is a perspective view illustrating an example of an appearance of an endoscope system according to a first embodiment.
Fig. 2 is a perspective view of a rigid portion illustrated in Fig. 1.
Fig. 3 is a perspective view in which an inside of the rigid portion illustrated in Fig. 2 is seen through.
Fig. 4 is an illustrative diagram illustrating an example of an assembly procedure of a compound-eye endoscope and a monocular endoscope.
Fig. 5 is an illustrative diagram illustrating an example of an assembly procedure of an image-capturing module.
Fig. 6 is an illustrative diagram illustrating an example of an assembly procedure of a 3D sub-frame.
Fig. 7 is perspective view of the 3D sub-frame to which an endoscope tip-end component is adhesively fixed.
Fig. 8 is an illustrative graph illustrating an example of a combination in which variation in characteristic evaluation values is suppressed.
Fig. 9 is an illustrative diagram illustrating an example of a combination of different technical performance evaluation characteristics.
Fig. 10 is a side cross-sectional view of the rigid portion illustrated in Fig. 2.
Fig. 11 is an exploded perspective view of the endoscope tip-end component and the image-capturing module illustrated in Fig. 3.
Fig. 12 is a flowchart illustrating an example of a procedure of a method of manufacturing an oblique viewing endoscope according to a first embodiment.
Fig. 13 is a flowchart illustrating an example of a procedure of 3D sub-frame adjustment.
Fig. 14 is a schematic diagram illustrating an example of a procedure of optical axis parallelism adjustment and image leveling adjustment of a 3D sub-frame.
Fig. 15 is a perspective view of an endoscope tip-end component to which a cover glass is adhesively fixed.
Fig. 16 is a perspective view of the image-capturing module before bending of a flexible substrate.
Fig. 17 is an illustrative diagram in which one image-capturing module is formed separately into a side-viewing endoscope and a direct-viewing endoscope.
Fig. 18 is a perspective view of an image-capturing module for side viewing in which a bending portion is in a bent state for a side-viewing endoscope.
Fig. 19 is a side view of the image-capturing module for side viewing illustrated in Fig. 18.
Fig. 20 is a front view of the image-capturing module for side viewing illustrated in Fig. 18.
Fig. 21 is a bottom view of the image-capturing module for side viewing illustrated in Fig. 18.
Fig. 22 is a perspective view of a rigid portion of a side-viewing endoscope in which the image-capturing module for side viewing is accommodated.
Fig. 23 is a cross-sectional view of the rigid portion of the side-viewing endoscope illustrated in Fig. 22.
Fig. 24 is a perspective view in which the rigid portion of the side-viewing endoscope illustrated in Fig. 22 is seen through.
Fig. 25 is a perspective view of the rigid portion of the side-viewing endoscope illustrated in Fig. 24 as viewed from a side opposite to the optical system.
Fig. 26 is a perspective view of an image-capturing module for direct viewing in which a bending portion is in a bent state for a direct-viewing endoscope.
Fig. 27 is a side view of the image-capturing module for direct viewing illustrated in Fig. 26.
Fig. 28 is a cross-sectional view of a rigid portion of a direct-viewing endoscope in which the image-capturing module for direct viewing is accommodated.
Fig. 29 is a perspective view of an image-capturing module for oblique viewing in which a bending portion is in a bent state for an oblique-viewing endoscope.
Fig. 30 is a side view of the image-capturing module for oblique viewing illustrated in Fig. 29.
Fig. 31 is a cross-sectional view of a rigid portion of an oblique-viewing endoscope in which the image-capturing module for oblique viewing is accommodated.

### DESCRIPTION OF EMBODIMENTS

### [Background to One Configuration of Present Disclosure]

A 3D endoscope capable of obtaining a captured image having left and right parallax and displaying the captured image stereoscopically is configured by assembling two image sensors and two lens barrels (optical systems) to one component. However, in the electronic endoscope apparatus disclosed in Patent Literature 1, a CCD module in which a CCD module for a left eye and a CCD module for a right eye are integrated is inserted into a hollow portion of a mounting member formed in a box shape, and is fixed by a screw, an adhesive, or the like. Therefore, there is a problem that the structure of the mounting member becomes complicated, which is not suitable for mass production, and the manufacturing cost of the 3D endoscope increases.

On the other hand, in the electronic endoscope apparatus disclosed in Patent Literature 1, the CCD module in which the CCD module for a left eye and the CCD module for a right eye are integrated is inserted into the hollow portion of the mounting member formed in a box shape, and is fixed by a screw, an adhesive, or the like. Therefore, the CCD module becomes a dedicated CCD module to be fixed to a mounting member for a direct-viewing endoscope, and in order to manufacture an endoscope for other applications (for example, an oblique-viewing endoscope or a side-viewing endoscope), a dedicated CCD module for the other applications has to be separately manufactured. There is a problem that the versatility is low, the CCD module is not suitable for mass production, and the manufacturing cost of the endoscope is high.

In the following embodiments, an example of a compound-eye endoscope or an endoscope that is devised in view of the above-described circumstances in the related art, is excellent in mass productivity, and can reduce the manufacturing cost will be described.

Hereinafter, embodiments in which a compound-eye endoscope and an endoscope according to the present disclosure is specifically disclosed will be described in detail with reference to the drawings as appropriate. Unnecessarily detailed description may be omitted. For example, detailed description of a well-known matter or repeated description of substantially the same configuration may be omitted. This is to avoid unnecessary redundancy in the following description and to facilitate understanding of those skilled in the art. The accompanying drawings and the following description are provided in order for those skilled in the art to fully understand the present disclosure, and are not intended to limit the subject matters described in the claims.

### [Configuration]

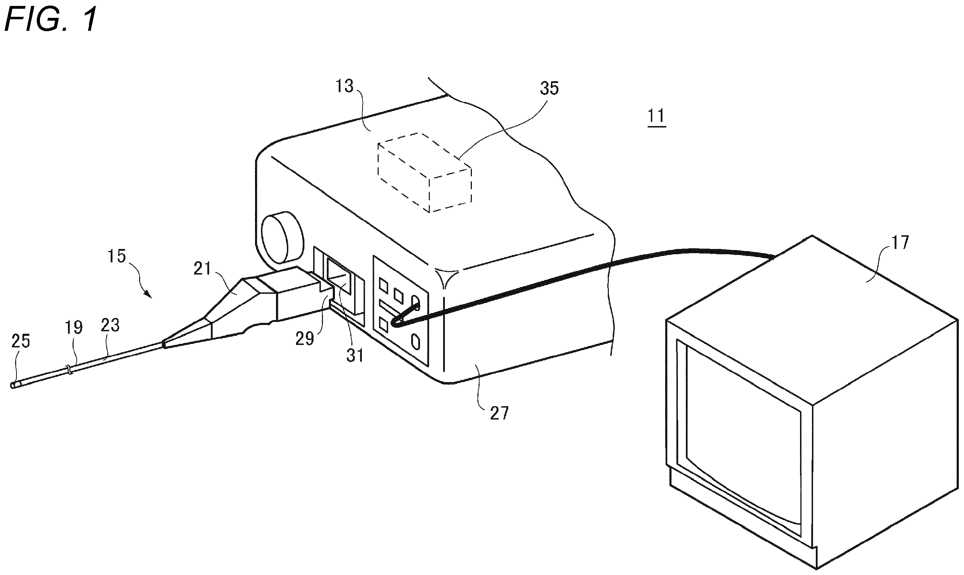

Fig. 1 is a perspective view illustrating an example of an appearance of an endoscope system 11 according to a first embodiment. As terms used here, an upward direction and a downward direction of a housing of a video processor 13 placed on a horizontal surface or a placement surface such as a horizontal table are referred to as "up" and "down", respectively. In addition, a side on which the endoscope captures an image of an observation target is referred to as "front (tip)", and a side connected to the video processor 13 is referred to as "rear".

The endoscope system 11 includes an oblique viewing endoscope 15 as an example of an endoscope, the video processor 13, and a monitor 17. The oblique viewing endoscope 15 used in the endoscope system 11 is an example. In addition to the oblique viewing endoscope 15, a direct viewing endoscope (may be referred to as a "direct-viewing endoscope") or a side viewing endoscope (may be referred to as a "side-viewing endoscope") may be used as an endoscope constituting the endoscope system 11. The oblique viewing endoscope 15 is, for example, a rigid endoscope or a flexible endoscope for medical use. The video processor 13 performs various types of image processing on a captured image (for example, including a still image and a moving image) obtained by capturing an image of an observation target (for example, an affected part) in a subject into which the oblique viewing endoscope 15 is inserted, and outputs the processed image. The monitor 17 displays an image in accordance with a display signal output from the video processor 13. Examples of the various types of image processing include, but are not limited to, color correction, gradation correction, and gain adjustment.

The oblique viewing endoscope 15 includes a scope 19 to be inserted into an observation target, and a plug portion 21 to which a rear end portion of the scope 19 is connected. The scope 19 includes a relatively long flexible portion 23 having flexibility, and a rigid portion 25 provided at a tip end of the flexible portion 23 and having rigidity. The oblique viewing endoscope 15 can be handled as a single finished product from the rigid portion 25 to the plug portion 21.

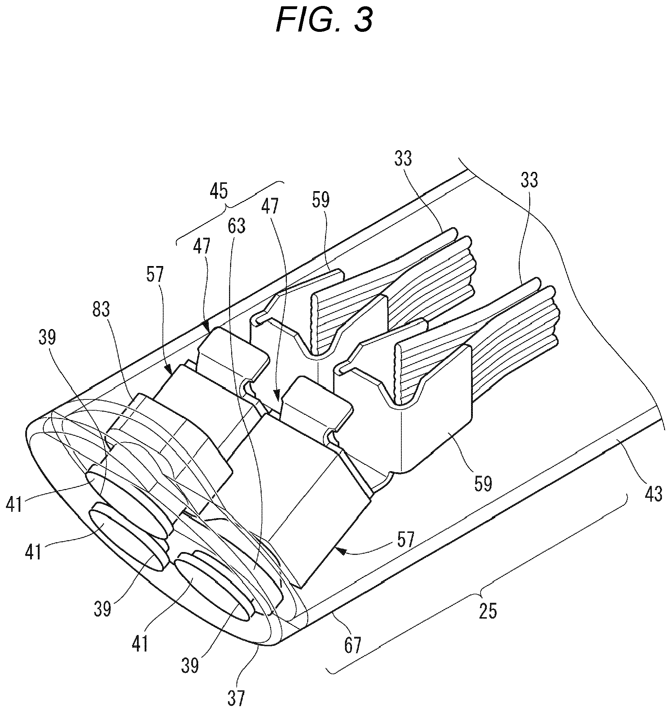

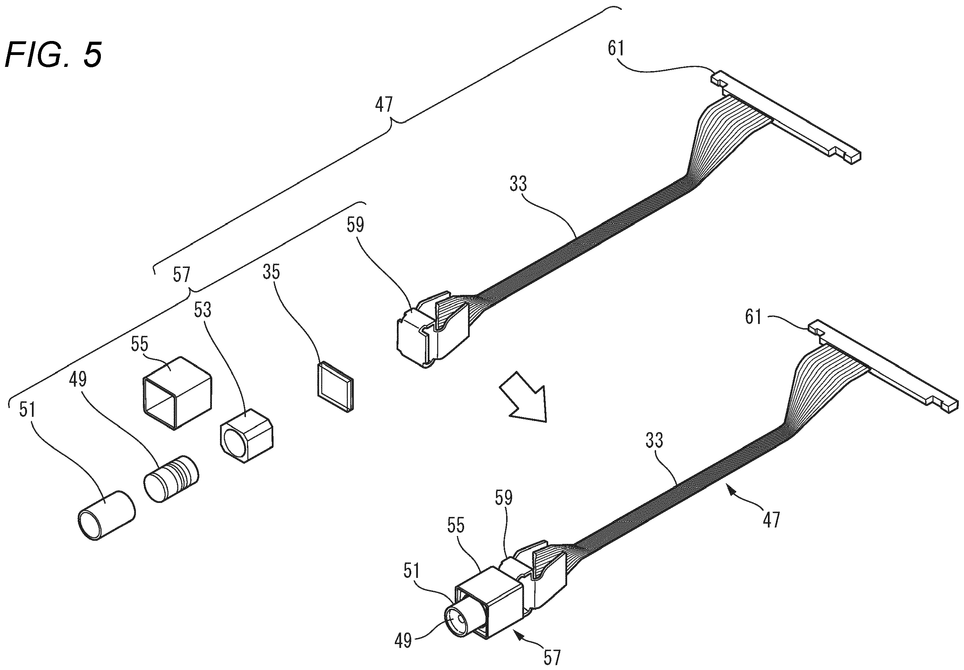

The video processor 13 includes a housing 27, performs various types of image processing on a captured image captured by the oblique viewing endoscope 15, and outputs a display signal after the image processing. A socket portion 31 into which a base end portion 29 of the plug portion 21 is inserted is provided on a front surface of the housing 27. When the plug portion 21 is inserted into the socket portion 31 and the oblique viewing endoscope 15 and the video processor 13 are electrically connected, transmission and reception of electric power and various signals (for example, a captured image signal or a control signal) between the oblique viewing endoscope 15 and the video processor 13 is possible. The electric power and various signals are transmitted from the plug portion 21 to the flexible portion 23 via a transmission cable 33 (see Fig. 3) inserted into the scope 19. A signal of a captured image output from an image sensor 35 (see Fig. 5) provided inside the rigid portion 25 is transmitted from the plug portion 21 to the video processor 13 via the transmission cable 33.

The video processor 13 performs various types of image processing (see the above description) on the signal of the captured image transmitted via the transmission cable 33, converts data of the captured image after the image processing into a display signal, and outputs the display signal to the monitor 17.

The monitor 17 is configured with a display device such as a liquid crystal display (LCD) or a cathode ray tube (CRT). The monitor 17 displays a captured image of an observation target captured by the oblique viewing endoscope 15. The monitor 17 displays, for example, a visible light image captured under illumination of, for example, visible light (that is, white light) for illuminating the observation target, which is guided from the video processor 13 to the rigid portion 25 via the plug portion 21.

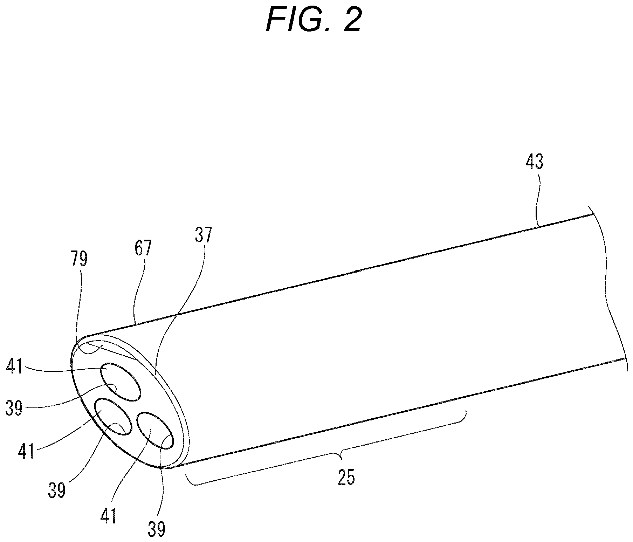

Fig. 2 is a perspective view of the rigid portion 25 illustrated in Fig. 1. In the oblique viewing endoscope 15, the rigid portion 25 includes an endoscope tip-end component 37. The endoscope tip-end component 37 can be formed of a metal having rigidity (for example, stainless steel) or a resin molded to have rigidity. The endoscope tip-end component 37 is formed in, for example, an elliptical plate shape. When the endoscope constituting the endoscope system 11 is a direct-viewing endoscope, the endoscope tip-end component 37 may be formed in a disc shape. As illustrated in Fig. 2, a plurality of window through holes 39 are provided in the endoscope tip-end component 37. In the present embodiment, three window through holes 39 are arranged in a radial direction from a center of the endoscope tip-end component 37. Each window through hole 39 is hermetically closed by adhesively fixing an objective cover glass 41. In the oblique viewing endoscope 15, the endoscope tip-end component 37 and a tip end portion of a sheath 43 adhesively fixed to the endoscope tip-end component 37 constitute the rigid portion 25 having a cylindrical appearance. In the oblique viewing endoscope 15 according to the present embodiment, a length of the rigid portion 25 is not limited to a length illustrated in Fig. 2, and is not particularly limited as long as the ease of inserting the oblique viewing endoscope 15 into a subject is satisfied.

Fig. 3 is a perspective view in which an inside of the rigid portion 25 illustrated in Fig. 2 is seen through. The oblique viewing endoscope 15 includes, inside the rigid portion 25, a 3D camera module 45 serving as an example of an endoscope module. A 3D sub-frame (hereinafter, referred to as "3D camera module 45") is configured with a plurality of image-capturing modules 47. In the present embodiment, the 3D camera module 45 is configured with, for example, two image-capturing modules 47 in order to capture images constituting a three-dimensional image as a 3D endoscope. The 3D camera module 45 is not limited to an example in which the 3D camera module 45 is configured with, for example, two image-capturing modules 47 in order to capture images constituting a three-dimensional image as a 3D endoscope. The 3D camera module 45 may be configured by combining image-capturing modules 47 having different optical filter characteristics, for example. That is, a compound-eye endoscope 101 to be described later is not limited to an endoscope having a function and an application as a 3D endoscope, and may be an endoscope that is configured by combining at least two image-capturing modules 47 having different optical filter characteristics and obtains an image other than a three-dimensional image, for example.

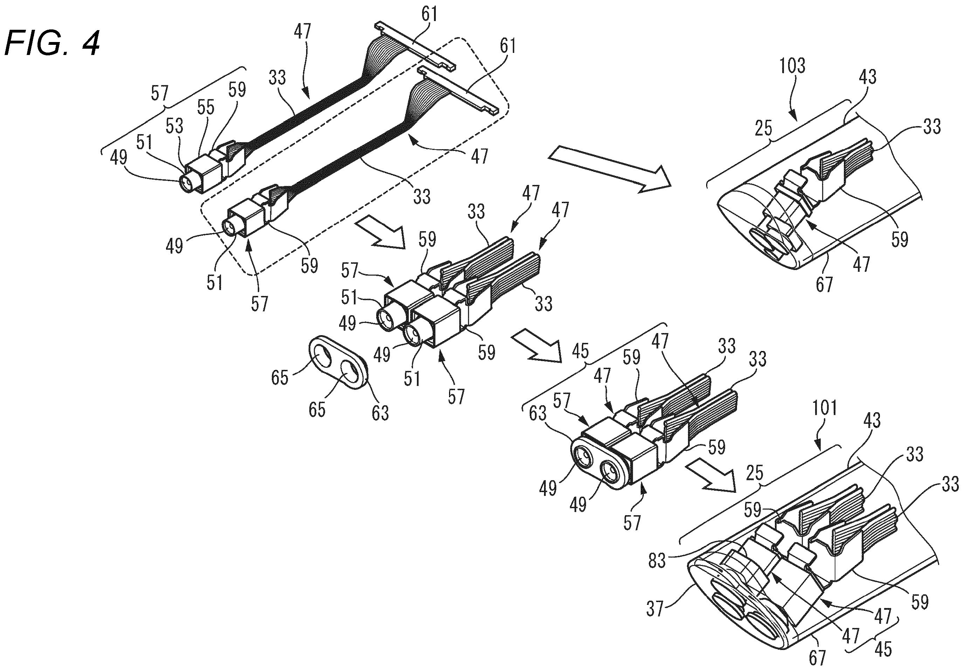

Fig. 4 is an illustrative diagram illustrating an example of an assembly procedure of the compound-eye endoscope 101 and a monocular endoscope 103. The 3D camera module 45 constitutes the compound-eye endoscope 101 by combining two image-capturing modules 47 formed in the same outer shape. On the other hand, a single image-capturing module 47 constitutes the monocular endoscope 103. The two image-capturing modules 47 having the same outer shape constitute one 3D camera module 45 by adhesively fixing respective lens barrels 51 to a pair of lens barrel insertion holes 65 formed in a sub-frame 63. The sub-frame 63 is formed of an elliptical plate material, and includes the pair of lens barrel insertion holes 65 spaced apart from each other in a longitudinal direction thereof. Each of the lens barrel insertion holes 65 is formed to be slightly larger than an outer diameter of the lens barrel 51. A gap that allows movement exists between the lens barrel 51 and the lens barrel insertion hole 65. In the present embodiment, a maximum allowable dimension of the lens barrel 51 is smaller than a minimum allowable dimension of the lens barrel insertion hole 65.

Accordingly, the two lens barrels 51 can move in a state of being inserted through the lens barrel insertion holes 65, and rotation adjustment around an optical axis of the optical system and swing adjustment around an axis orthogonal to the optical axis are possible for the two lens barrels 51 in a state of being inserted through the lens barrel insertion holes 65. The two image-capturing modules 47, which are positioned relative to each other and positioned relative to the sub-frame 63, and the sub-frame 63 are assembled to form the 3D camera module 45 in such a manner that an outer periphery of each lens barrel 51 is adhesively fixed to an inner periphery of each lens barrel insertion hole 65 by an adhesive 81. That is, the image-capturing module 47 is assembled as the monocular endoscope 103 by being incorporated into the rigid portion 25 as a single body. In addition, the image-capturing module 47 is assembled as the compound-eye endoscope 101 by incorporating the 3D camera module 45, in which the image-capturing module 47 is combined with the sub-frame 63, into the rigid portion 25. It is also possible to implement an adjustment mechanism in which the sub-frame 63 adopts a material that is easily deformed, such as aluminum, and after the image-capturing module 47 is fixed, the image-capturing module 47 is adjusted by being bent and twisted to be permanently deformed.

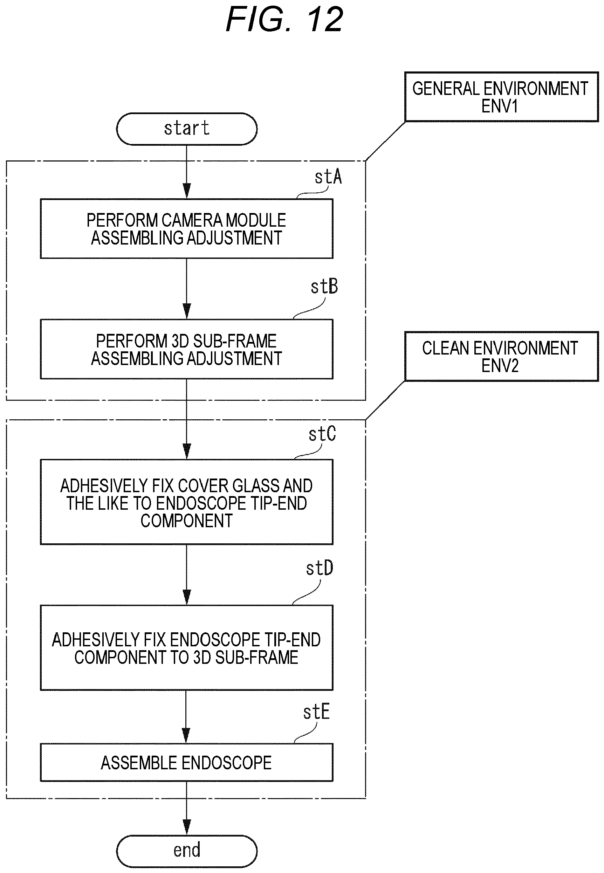

Fig. 5 is an illustrative diagram illustrating an example of an assembly procedure of the image-capturing module 47. In the image-capturing module 47, the lens barrel 51 that houses a lens 49 that is an optical system, the image sensor 35, and a sensor holding member 53 that relatively fixes the lens barrel 51 and the image sensor 35, are assembled in a first atmosphere ENV1 (see Fig. 12). Here, the first atmosphere is, for example, an environment clean enough to allow assembling of a precision electronic component (in other words, a general assembly work environment). An outside of the sensor holding member 53 is further covered with a rectangular cylindrical cover 55. The lens 49, the lens barrel 51, the image sensor 35, the sensor holding member 53, the cover 55, and a flexible substrate 59 (described later) constitute a camera portion 57. The image-capturing module 47 including the camera portion 57 is a camera module that can operate alone.

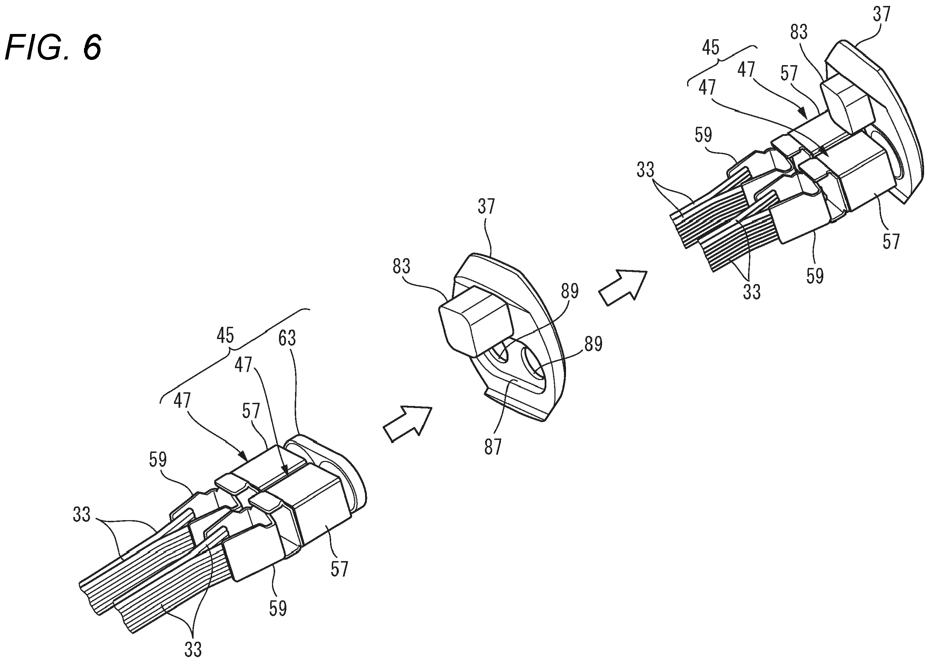

Fig. 6 is an illustrative diagram illustrating an example of an assembly procedure of the 3D sub-frame. The 3D sub-frame (that is, the 3D camera module 45) in which the two image-capturing modules 47 are fixed by the sub-frame 63 is adhesively fixed to a sub-frame fixing recess 87 of the endoscope tip-end component 37. The sub-frame fixing recess 87 is formed of an elliptical recess into which the sub-frame 63 formed of an elliptical plate member is fitted, and is formed to have a depth of about a plate thickness of the sub-frame 63. The 3D camera module 45 and the endoscope tip-end component 37 can be assembled without requiring fine adjustment. That is, the 3D camera module 45 and the endoscope tip-end component 37 can be assembled with mechanical fitting (fitting) accuracy of the sub-frame 63 and the sub-frame fixing recess 87. In the 3D camera module 45 in which the sub-frame 63 is fixed to the sub-frame fixing recess 87, each of the lens barrels 51 enters a lens barrel insertion hole 89 of the endoscope tip-end component 37 and is positioned coaxially with the lens barrel insertion hole 89.

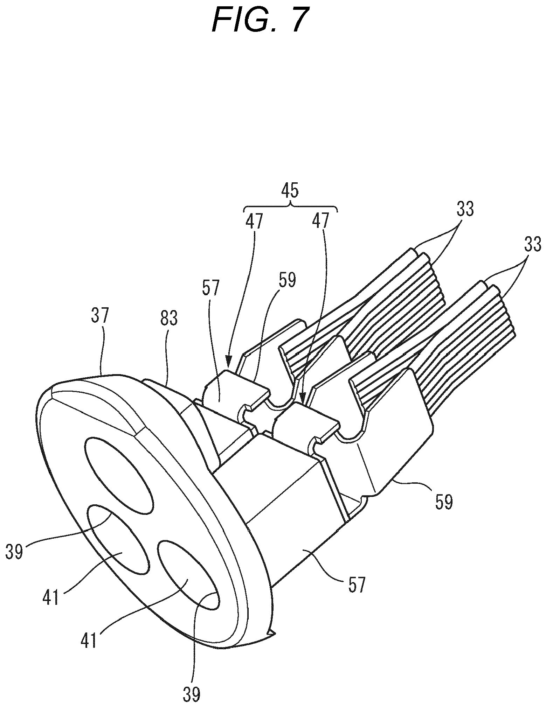

Fig. 7 is perspective view of the 3D sub-frame to which the endoscope tip-end component 37 is adhesively fixed. In the 3D camera module 45 integrally assembled to the endoscope tip-end component 37, each lens barrel 51 is disposed on the back of the cover glass 41 fitted to each of the two window through holes 39 of the endoscope tip-end component 37. A light source 83 is disposed on the back of the cover glass 41 fitted to the remaining one of the three window through holes 39 formed in the endoscope tip-end component 37.

The camera portion 57 has a plurality of pads (not shown) on a back surface of the image sensor 35. The flexible substrate 59 is conductively connected to the back surface of the image sensor 35 via the pad. The flexible substrate 59 is disposed between the image sensor 35 and the transmission cable 33. As the transmission cable 33, for example, a flat cable in which a plurality of parallel insulated conductors are formed in a band shape on the same plane is used. A transmission circuit in which a plurality of linear conductors are pattern-printed is formed on the flexible substrate 59. The flexible substrate 59 conductively connects each electric wire of the transmission cable 33 to the transmission circuit. Accordingly, the image sensor 35 is connected to the transmission cable 33 via the flexible substrate 59. As the flexible substrate 59, for example, a flexible flat cable (FFC) formed in a flexible band-shaped cable by covering a conductor formed of a plurality of band-shaped thin plates with an insulating sheet material, or a flexible printed wiring board (FPC) in which a linear conductor is pattern-printed on a flexible insulating substrate can be used.

A connector 61 is connected to a base end of the transmission cable 33. The connector 61 is accommodated in the plug portion 21 (see Fig. 1) and can be electrically connected to the socket portion 31. The camera portion 57, the transmission cable 33, and the connector 61 constitute one image-capturing module 47.

As described above, the compound-eye endoscope 101 includes the lens barrel 51 that houses the optical system, the image sensor 35, and the sensor holding member 53 that relatively fixes the lens barrel 51 and the image sensor 35. The compound-eye endoscope 101 includes two or more image-capturing modules 47 formed in the same outer shape, a sub-frame 63 that relatively fixes each of the two or more image-capturing modules 47, and an outer shell 67 that accommodates and fixes the sub-frame 63 and the two or more image-capturing modules 47.

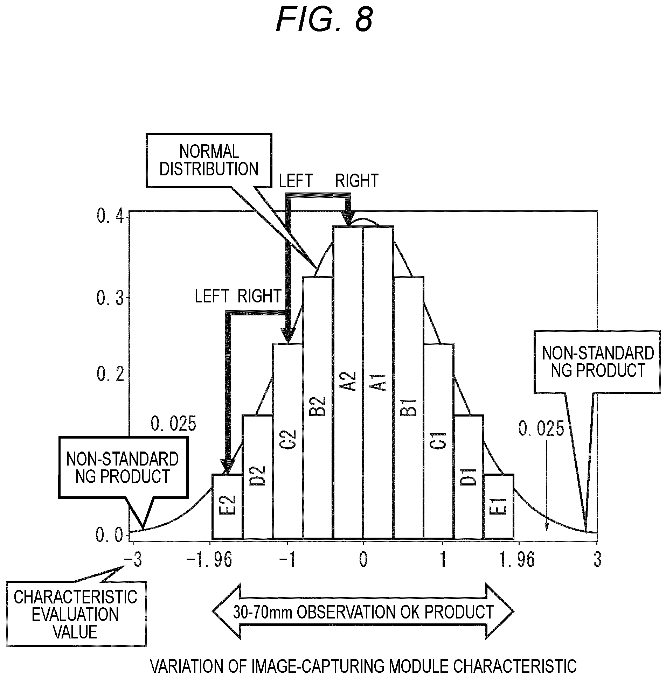

Fig. 8 is an illustrative graph illustrating an example of a combination in which variation in characteristic evaluation values is suppressed. Here, the two or more image-capturing modules 47 have performance evaluation characteristics including an outer shape that satisfy a predetermined correlation, as a result of a technical performance evaluation test for evaluating various performance evaluation characteristics including 3D image-capturing performance measured in advance before shipment of the 3D camera module 45.

Items of the performance evaluation characteristics to be measured in the technical performance evaluation test include, for example, a best focus position (in other words, an observation range) related to the 3D image-capturing performance, a degree of one-sided blur, an image brightness, a hue (variation in individual difference of the image sensor 35), sensitivity (variation in individual difference of the image sensor 35), a peripheral light amount, resolution performance, and the like. The best focus position (in other words, the observation range) is a performance evaluation characteristic related to the 3D image-capturing performance, and a range that can be observed by the image-capturing module 47 varies to some extent due to an adjustment error between the image sensor 35 and the lens barrel 51. The degree of one-sided blur indicates an error caused by an optical axis not being perpendicular to a sensor surface of the image sensor 35 when the optical axis is formed by aligning the optical system (for example, the lens 49). The image brightness is generated due to, for example, deviation of an aperture in the optical system (for example, the lens 49). The hue (the variation in individual difference of the image sensor 35) indicates a hue caused by the individual difference of the image sensor 35. The sensitivity (sensor variation) also indicates variation in output caused by the individual difference of the image sensor 35. The peripheral light amount is a light amount obtained at a corner of an angle of view with respect to a center of the angle of view. The resolution performance mainly depends on an individual difference in the lens optical system inside the lens barrel.

In Fig. 8, for example, variation in a characteristic evaluation value (that is, a value indicated by the performance evaluation characteristics) of the best focus position (in other words, the observation range) among the performance evaluation characteristics is taken on the horizontal axis, and a probability density is taken on the vertical axis. For the performance evaluation characteristic, a difference with respect to target performance is minimum at the center of the horizontal axis. A rank is classified by applying an arbitrary width to this difference. In Fig. 8, the number of samples for each rank in the performance evaluation characteristic (for example, the best focus position (in other words, the observation range)) obtained from a predetermined amount of single samples of the image-capturing module 47 is represented by a probability distribution curve (normal distribution). In the variation of the characteristic evaluation value, the image-capturing module 47 having a high evaluation occupies a majority, and the larger the difference in the evaluation characteristic is, the smaller the variation becomes, and the others become non-standard NG products. As for the best focus position of the single sample of image-capturing module, for example, a range of 30 mm to 70 mm is assumed to be that of a standard non-defective product (observation OK product).

Since the image-capturing module 47 is manufactured with a tolerance, an actual best focus position is in the range of 30 mm to 70 mm described above. When a median value of this range is, for example, 50 mm, the range can be set to 50 mm ± 10 mm. When the median value of 50 mm is considered as a reference point of the characteristic evaluation value, an allowable range of the characteristic evaluation value can be set to the value of the reference point (reference value) ± 20%. That is, a rank whose characteristic evaluation value is less than [the reference value of the characteristic evaluation value - 20% of the reference value] is a non-standard NG product, and a rank whose characteristic evaluation value is greater than [the reference value of the characteristic evaluation value + 20% of the reference value] is non-standard NG product.

Therefore, the predetermined correlation of the performance evaluation characteristics of each of the two image-capturing modules 47 constituting the compound-eye endoscope 101 can be, for example, a relationship in which the variation in the characteristic evaluation value is suppressed (that is, a relationship in which the variation in the characteristic evaluation value is within a predetermined range).

In the compound-eye endoscope 101, the characteristic variation of the left and right image-capturing modules 47 is suppressed, so that a user is less likely to feel fatigue even when using the compound-eye endoscope 101 for a long time. For example, in a case of a combination of a product of rank C2 and a product of rank A1 illustrated in Fig. 8, a feeling of discomfort in the 3D vision is caused and a feeling of fatigue is increased. Therefore, for example, the 3D camera module 45 is assembled by combining "a product of right C2 and a product of left E2" or "a product of left C2 and a product of right A2". That is, products whose variation in the characteristic evaluation value is suppressed are used. Accordingly, it is possible to obtain the compound-eye endoscope 101 with which the user is less likely to feel fatigue even when using the compound-eye endoscope 101 for a long time.

A combination interval (range of variation) and the left and right (positive and negative) are not limited thereto. Although the best focus position is taken as an example in the above description, the characteristic evaluation value may be a characteristic evaluation value corresponding to any performance evaluation characteristic of the degree of one-sided blur, the image brightness, the hue (sensor variation), the sensitivity (sensor variation), the peripheral light amount, and the resolution performance described above.

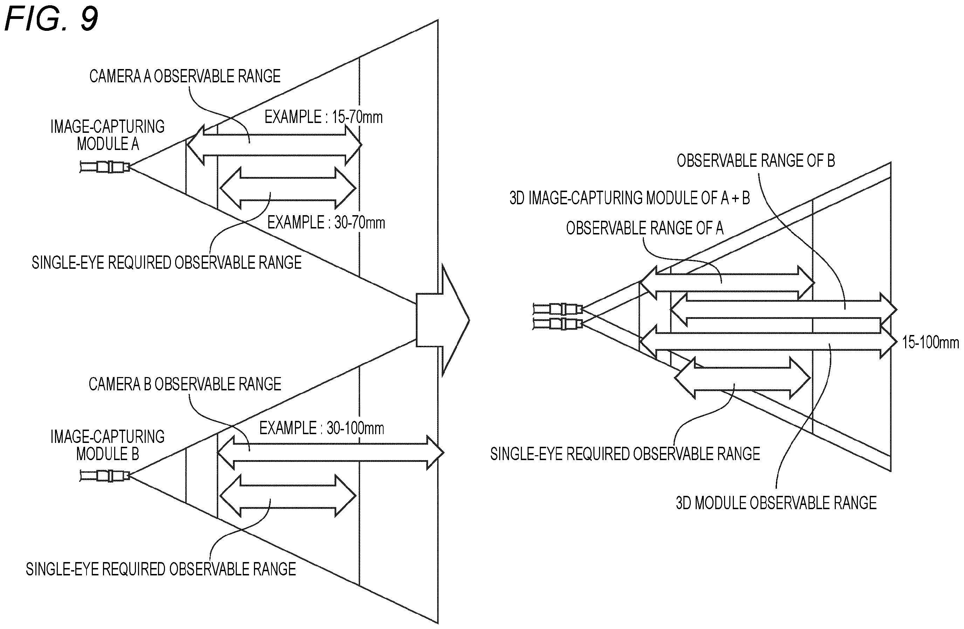

Fig. 9 is an illustrative diagram illustrating an example of a combination of different technical performance evaluation characteristics. In addition, the predetermined correlation may be, for example, a relationship in which the respective image-capturing modules 47 have different technical performance evaluation characteristics, in addition to the relationship in which the variation in the characteristic evaluation value is suppressed (that is, the relationship in which the variation in the characteristic evaluation value is within a predetermined range). Here, as the different technical performance evaluation characteristics, for example, those in which the respective image-capturing modules 47 have different focal depths (actual focal positions) can be cited.

In this case, in an image-capturing module X, an observable range of a camera A corresponding to the image-capturing module X is, for example, 15 mm to 70 mm. A singleeye required observation range is, for example, 30 mm to 70 mm. In an image-capturing module Y, an observable range of a camera B corresponding to the image-capturing module Y is, for example, 30 mm to 100 mm. In this case, an observable range of the 3D camera module 45 configured with the camera A and the camera B is 15 mm to 100 mm. In the compound-eye endoscope 101 configured using the two image-capturing modules (the camera A and the camera B) having such a correlation, images captured by the left and right image-capturing modules 47 complement each other in an image synthesized and recognized by brain processing of the user (human), and thus a wide recognition acceptance range can be obtained.

The 3D camera module 45 includes the two image-capturing modules 47 and the sub-frame 63 that relatively fixes the two image-capturing modules 47. The sub-frame 63 and the two image-capturing modules 47 are accommodated in an outer shell of a main body of the oblique viewing endoscope 15 that is assembled in a second atmosphere different from the first atmosphere (more specifically, a second atmosphere that is a cleaner environment than the first atmosphere). Here, the second atmosphere is an assembly work environment set to a higher degree of cleanliness than the first atmosphere. The work of accommodating the 3D camera module 45 into the outer shell in the second atmosphere is performed in a limited clean process that receives official approval, for example.

The sub-frame 63 has a pair of lens barrel insertion holes 65 into which the two lens barrels 51 are loosely fitted, respectively. In each of the pair of image-capturing modules 47, each outer periphery of the two lens barrels 51 positioned relative to each other is adhesively fixed to the inner periphery of the lens barrel insertion hole 65.

The 3D camera module 45 can capture images constituting a three-dimensional image, with at least two of the two or more image-capturing modules 47 thereof.

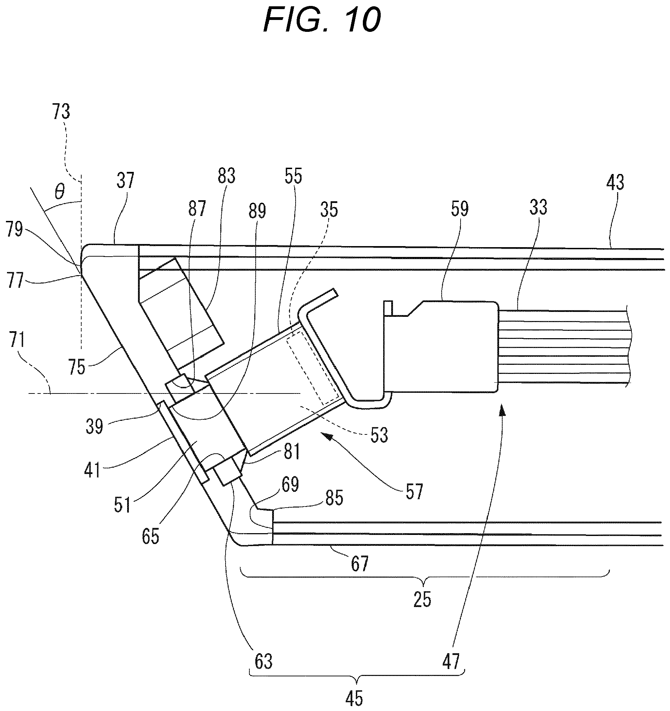

Fig. 10 is a side cross-sectional view of the rigid portion 25 illustrated in Fig. 2. The outer shell 67 as an example of an outer shell portion of the oblique viewing endoscope 15 includes the endoscope tip-end component 37 to which the sub-frame 63 is fixed, and the sheath 43 in which a tubular tip end opening 69 is closed by the endoscope tip-end component 37. The plug portion 21 (see Fig. 1) is attached to a rear end portion of the sheath 43. The connector 61 conductively connected to the base end of the transmission cable 33 is accommodated in the plug portion 21.

The sheath 43 is made of a material having flexibility, and covers an outer periphery of the flexible portion 23 and an outer periphery of a part of the rigid portion 25 of the oblique viewing endoscope 15. The tip end opening 69 of the sheath 43 is inclined by a predetermined angle θ with respect to a virtual plane 73 perpendicular to an axis 71 of the rigid portion 25 and is opened. The endoscope tip-end component 37 is inclined with respect to the virtual plane 73 to close the tip end opening 69. In the endoscope tip-end component 37, a chamfered portion 79 from which an edge is removed is formed at a forward-inclined tip end portion 77 of the inclined tip end surface 75 (see Fig. 2). The sub-frame 63 and the endoscope tip-end component 37 are adhesively fixed to each other by the adhesive 81.

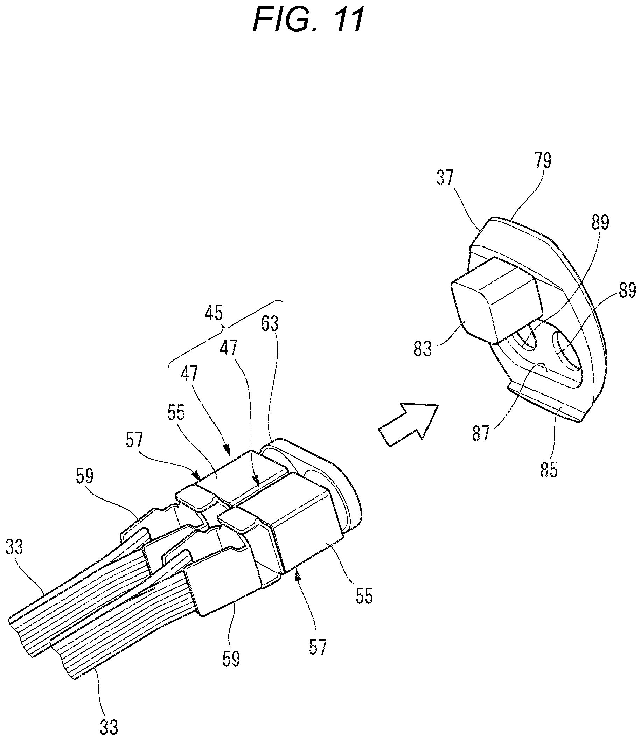

Fig. 11 is an exploded perspective view of the endoscope tip-end component 37 and the image-capturing module 47 illustrated in Fig. 3. A plurality of window through holes 39 are provided in the endoscope tip-end component 37 (see Fig. 2). In the present embodiment, three window through holes 39 corresponding to two image-capturing modules 47 and one light source 83 are provided. Each window through hole 39 is hermetically closed by adhesively fixing the objective cover glass 41. A sheath fixing portion 85 protrudes from a lower back surface of the endoscope tip-end component 37 along the axis 71 of the rigid portion 25. An inner periphery of the sheath 43 is fixed to an outer periphery of the sheath fixing portion 85.

The endoscope tip-end component 37 has the sub-frame fixing recess 87 for relatively positioning and fixing the sub-frame 63. The sub-frame fixing recess 87 is an elliptical recess elongated in a direction in which the two image-capturing modules 47 are arranged side by side. The lens barrel insertion holes 89 into which the lens barrels 51 of the two image-capturing modules 47 are individually inserted are provided in the bottom of the sub-frame fixing recess 87. That is, as illustrated in Fig. 10, the sub-frame 63 is adhesively fixed to the sub-frame fixing recess 87, and at the same time, the lens barrel 51 fixed to the lens barrel insertion hole 65 of the sub-frame 63 and protruded is adhesively fixed to the lens barrel insertion hole 89 of the endoscope tip-end component 37. The cover glass 41 is disposed in front of the lens barrel 51.

In the oblique viewing endoscope 15, the light source 83 that emits illumination light to the outside of outer shell 67 is disposed on a back surface of at least one cover glass 41. As the light source 83, for example, a light emission diode (LED) as an example of a point light source can be suitably used. The LED is adhesively fixed to the endoscope tip-end component 37 so as to coincide with a light emitting portion on the back of one of the three window through holes 39. The light source 83 may include not only an LED but also a cooling mechanism (not shown) as a measure against heat dissipation during irradiation of light from the LED.

It can be said that oblique viewing endoscope 15 having the above configuration has the following structure in terms of structure. That is, the structure of the oblique viewing endoscope 15 includes two or more image-capturing modules 47. Each image-capturing module 47 includes the lens barrel 51 housing an optical system, the image sensor 35, and the sensor holding member 53 that relatively fixes the lens barrel 51 and the image sensor 35. The sub-frame 63 that relatively fixes each of the two or more image-capturing modules 47 is provided in the outer shell of the main body of the oblique viewing endoscope 15.

In the structure of the oblique viewing endoscope 15, the sub-frame 63 is isolated from the outside of the outer shell 67. Here, being isolated means that the sub-frame 63 is completely covered without having a portion thereof appearing outside the outer shell 67.

### [Manufacturing Method]

Next, a method of manufacturing the oblique viewing endoscope 15 according to the first embodiment will be described.

Fig. 12 is a flowchart illustrating an example of a procedure of a method of manufacturing the oblique viewing endoscope 15 according to the first embodiment. The manufacturing of the oblique viewing endoscope 15 is performed separately in a first atmosphere that is a general environment and in a second atmosphere which is a more clean environment than the general environment.

In Fig. 12, in the general environment, assembling adjustment of the image-capturing module 47 (step stA shown in Fig. 12) and assembling adjustment of the 3D camera module 45 (step stB) are performed. That is, in the first atmosphere ENV1, a step of assembling the image-capturing module 47 by relatively fixing the lens barrel 51 housing an optical system and the image sensor 35 by using the sensor holding member 53 (step stA), and a step of relatively fixing each of the two or more image-capturing modules 47 using the sub-frame 63 (step stB) are performed.

On the other hand, in a clean environment, adhesion and fixation of the cover glass 41 and the like to the endoscope tip-end component 37 (step stC), adhesion and fixation of the sub-frame 63 and the endoscope tip-end component 37 (step stD), and endoscope assembling in which the sheath 43 is fixed to the endoscope tip-end component 37 to which the sub-frame 63 is fixed (step stE) are performed. That is, in the second atmosphere ENV2 that is more clean than the first atmosphere ENV1, a step of adhesively fixing the cover glass 41 to the endoscope tip-end component 37 (step stC), a step of fixing the sub-frame 63 to the back surface of the endoscope tip-end component 37 (step stD), and a step of forming the outer shell 67 of the endoscope main body by fixing the endoscope tip-end component 37 to the tip end opening 69 of the tubular sheath 43, and at the same time, hermetically sealing the sub-frame 63 and the two or more image-capturing modules 47 in the outer shell (step stE) are performed.

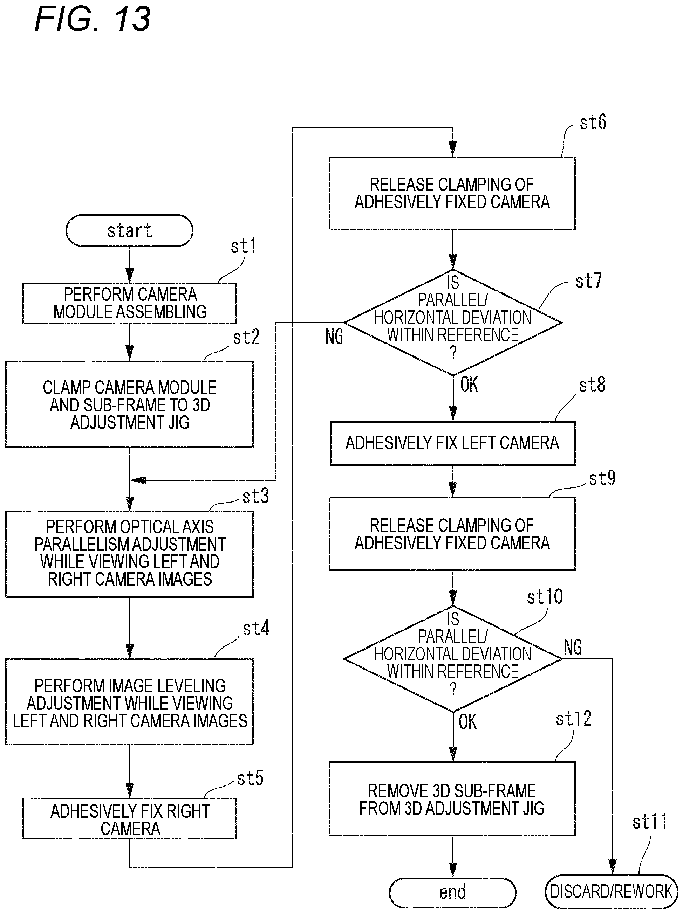

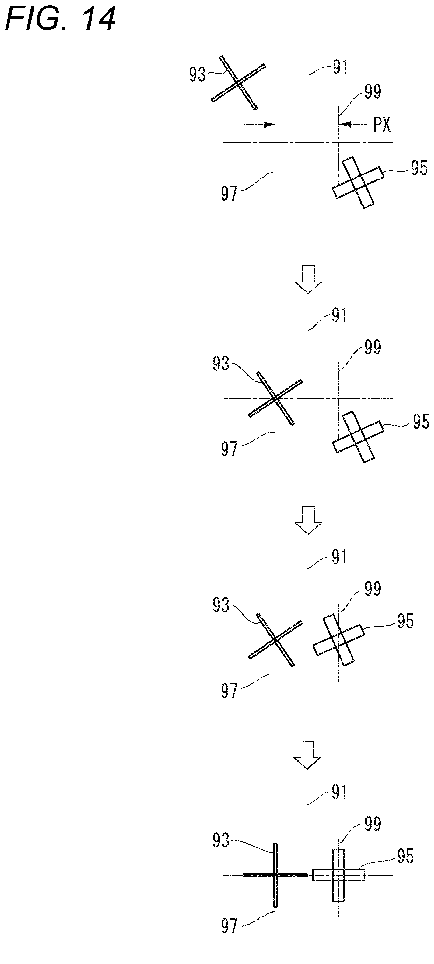

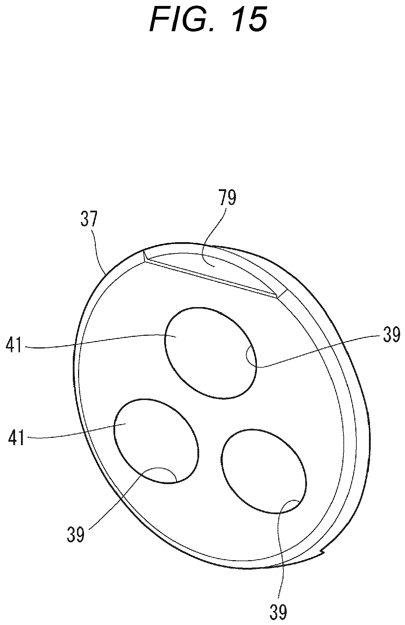

Fig. 13 is a flowchart illustrating an example of a procedure of 3D sub-frame adjustment. Fig. 14 is a schematic diagram illustrating a procedure of optical axis parallelism adjustment and image leveling adjustment of the 3D camera module 45. Fig. 15 is a perspective view of the endoscope tip-end component 37 to which the cover glass 41 is adhesively fixed. In the description of Fig. 13, Figs. 9 to 12 are referred to as necessary.

In Fig. 13, in the assembling adjustment of the image-capturing module 47 (step stA), when the assembly of the 3D camera module 45 is started (st1, see Fig. 13), first, the two image-capturing modules 47 and the sub-frame 63 are clamped (fixed) to a 3D adjustment jig (not shown) (st2).

In Fig. 14, an image obtained by capturing an image of a target 91 is acquired from each of the two image-capturing modules 47 (that is, the left camera and the right camera) clamped to the 3D adjustment jig. In Fig. 14, an image of the target 91 is indicated by a cross mark of an alternate long and short dash line. A left camera image 93 representing a direction and a rotation state of an optical axis of the left camera is indicated by a thin cross mark. A right camera image 95 representing a direction and a rotation state of an optical axis of the right camera is indicated by a thick cross mark.

In this adjustment procedure, first, an operator performs the optical axis parallelism adjustment while viewing the left and right camera images (st3). For example, the optical axis of the left camera image 93 is made aligned with a left scale 97 of the target 91. Next, the optical axis of the right camera image 95 is made aligned with a right scale 99 of the target 91. The left scale 97 and the right scale 99 are set at the same distance from a center of the target in a horizontal direction. A distance between the left and right scales is parallax px.

Next, the operator performs the image leveling adjustment while viewing the left and right camera images (st4). The image leveling adjustment is performed by rotating the left and right cameras around the respective optical axes. After the optical axis parallelism adjustment and the image leveling adjustment are completed, for example, the right camera (that is, one image-capturing module 47) is adhesively fixed to the sub-frame 63 by the operator (st5).

Next, the operator releases the clamping of the right camera that is adhesively fixed (st6). It is determined again whether deviation of the optical axis parallelism and the image leveling is within a reference in a state where the clamping of the right camera is released (st7). When the deviation is not within the reference in the state of the clamping being released, the adjustment is performed again from the optical axis parallelism adjustment. When the deviation is within the reference even in the state of the clamping being released, the left camera (that is, the other image-capturing module 47) is adhesively fixed to the sub-frame 63 (st8).

Next, the operator releases the clamping of the left camera that is adhesively fixed (st9). It is determined again whether the deviation of the optical axis parallelism and the image leveling is within the reference (st10). When the deviation is not within the reference in the state of the clamping being released, a workpiece (that is, the right camera and the left camera under working) is discarded or recycled (st11). When the deviation is within the reference even in the state of the clamping being released, a completed workpiece is removed from the 3D adjustment jig (st12), and the assembling adjustment of the 3D camera module 45 in the first atmosphere, which is a general environment, is completed (stB).

On the other hand, in the second atmosphere that is a clean environment, the cover glass 41 and the like are adhesively fixed to the window through hole 39 of the endoscope tip-end component 37 by the operator (stC).

Next, the endoscope tip-end component 37 is adhesively fixed to the sub-frame 63 of the 3D camera module 45 by the operator (stD). The endoscope tip-end component 37 is adhesively fixed in a state where an outer periphery of the 3D camera module 45 is inserted into the sub-frame fixing recess 87 (see Fig. 11). When the 3D camera module 45 is to be brought into the second atmosphere ENV2, the 3D camera module 45 may be subjected to sterilization treatment as necessary.

Finally, the operator performs the endoscope assembling in which the sheath 43 is fixed to the endoscope tip-end component 37 to which the sub-frame 63 is fixed (stE). In the endoscope assembling, the sheath 43, inside which the transmission cable 33 passes, is fed to the endoscope tip-end component 37, and the tip end opening 69 of the sheath 43 is joined to an outer periphery of the endoscope tip-end component 37, so that the sealing of the rigid portion 25 in the second atmosphere is completed.

The connector 61 is connected to the transmission cable 33 led out from a base end of the sheath 43. The plug portion 21 accommodating the connector 61 is attached to the base end of the sheath 43. Accordingly, the manufacturing of oblique viewing endoscope 15 is completed.

### [Application Example]

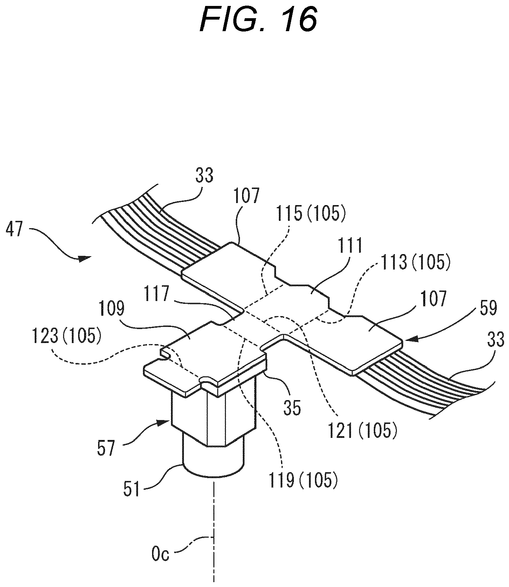

Fig. 16 is a perspective view of the image-capturing module 47 before bending of the flexible substrate 59. In the endoscope according to the first embodiment, the flexible substrate 59 provided in the image-capturing module 47 can be bent at a plurality of bending portions 105. The flexible substrate 59 is formed in a T shape by a pair of both wing portions 107 in a band plate shape to which the transmission cable 33 formed of a flat cable is connected at both ends, and a leg-like portion 109 extending in an orthogonal direction from a center in a longitudinal direction of the pair of both wing portions 107. In the T-shaped flexible substrate 59, a plurality of linear conductors having the leg-like portion 109 as one end and the pair of both wing portions 107 as the other end are embedded in parallel. An end portion of each linear conductor is exposed as a land on at least one of front and back surfaces of the leg-like portion 109 and both wing portions 107.

In the flexible substrate, the pair of both wing portions 107 are connected to a pair of parallel edge portions of a quadrangular both-wing connection portion 111 respectively. The leg-like portion 109 is connected to one another edge portion of the both-wing connection portion 111. The flexible substrate 59 includes a linear first side-portion bending portion 113 and a linear second side-portion bending portion 115 between the pair of both wing portions 107 and the both-wing connection portion 111. In the flexible substrate 59, a rectangular neck portion 117 is formed between the both-wing connection portion 111 and the leg-like portion 109. The flexible substrate 59 has a first neck-portion bending portion 119 and a second neck-portion bending portion 121, which are linear and parallel, between the neck portion 117 and the leg-like portion 109 and between the neck portion 117 and the both-wing connection portion 111. In addition, the leg-like portion 109 has, at an extending tip end, a linear tip-end bending portion 123 parallel to the first neck-portion bending portion 119. The first side-portion bending portion 113, the second side-portion bending portion 115, the first neck-portion bending portion 119, the second neck-portion bending portion 121, and the tip-end bending portion 123 constitute a bending portion 105 that can be bent on both the front and back sides of the flexible substrate 59.

The image sensor 35 is conductively connected to one surface of the leg-like portion 109 via a pad. The plurality of linear conductors connected to the pad are conductively connected to at least one of the two transmission cables 33 at the pair of both wing portions 107.

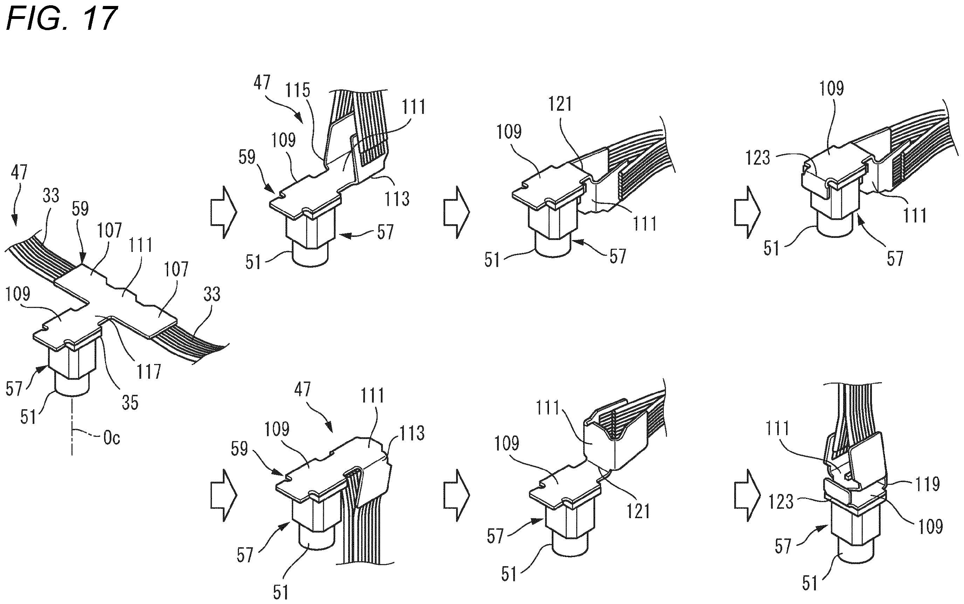

Fig. 17 is an illustrative diagram in which one image-capturing module 47 is formed separately into a side-viewing endoscope 125 and a direct-viewing endoscope 127. The creation example of Fig. 17 is an example, and the image-capturing module 47 can be separately created as an oblique-viewing endoscope 129. In the flexible substrate 59, the first side-portion bending portion 113, the second side-portion bending portion 115, the first neck-portion bending portion 119, the second neck-portion bending portion 121, and the tip-end bending portion 123 are appropriately bent, so that it is possible to variously change a direction of an optical axis Oc of the optical system and a lead-out direction of the transmission cable 33.

When the endoscope is incorporated into the rigid portion 25 having rigidity and provided at the tip end of the flexible portion 23, the bending portion 105 of the flexible substrate 59 is in a bent state according to a shape of the rigid portion 25.

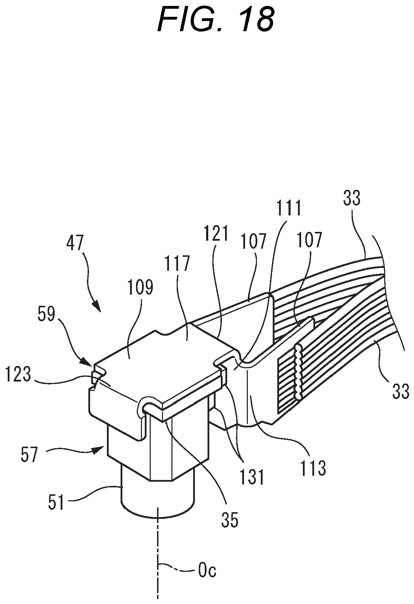

Fig. 18 is a perspective view of the image-capturing module 47 for side viewing in which the bending portion 105 is in a bent state for a side-viewing endoscope. In the image-capturing module 47 for side viewing, in the T-shaped image-capturing module 47, the both wing portions 107 are bent in parallel at the first side-portion bending portion 113 and the second side-portion bending portion 115 with respect to the both-wing connection portion 111. In this case, the leg-like portion 109 is bent at a right angle to the both-wing connection portion 111 at the second neck-portion bending portion 121. In addition, the leg-like portion 109 is bent, at the tip-end bending portion 123, in the same direction as the both-wing connection portion 111 to be in parallel with the both-wing connection portion 111.

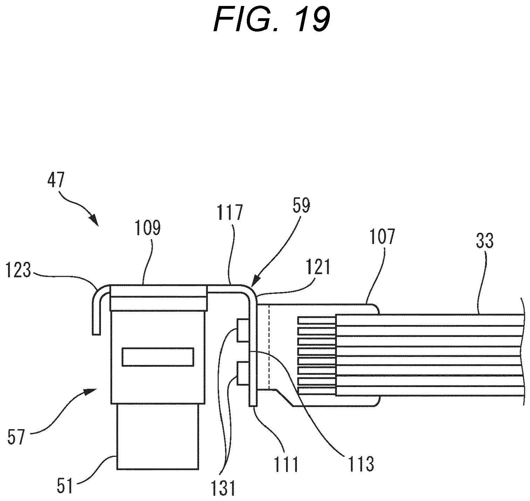

Fig. 19 is a side view of the image-capturing module 47 for side viewing illustrated in Fig. 18. In the image-capturing module 47 for side viewing, the camera portion 57 is orthogonal to an extending direction of the transmission cable 33 over an entire length in a direction along the optical axis Oc. An electronic component 131 such as a resistor or a capacitor is mounted on a surface of the both-wing connection portion 111 facing the camera portion 57.

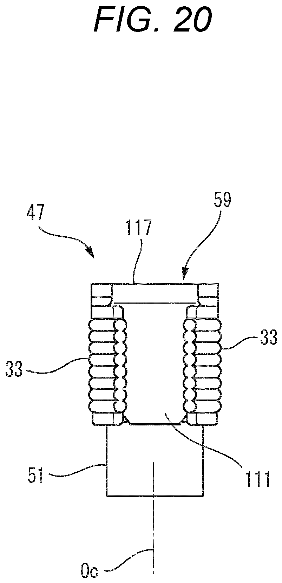

Fig. 20 is a front view of the image-capturing module 47 for side viewing illustrated in Fig. 18. In the image-capturing module 47 for side viewing, a tip end of the lens barrel 51 in the direction of the optical axis Oc protrudes outward from the both-wing connection portion 111.

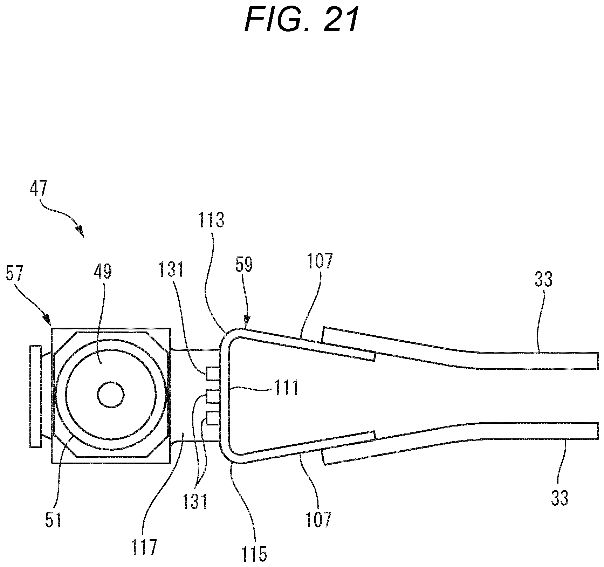

Fig. 21 is a bottom view of the image-capturing module 47 for side viewing illustrated in Fig. 18. In the image-capturing module 47 for side viewing, a separation gap between the pair of transmission cables 33 falls within a range of a length of one side portion of the both-wing connection portion 111 having a substantially square shape.

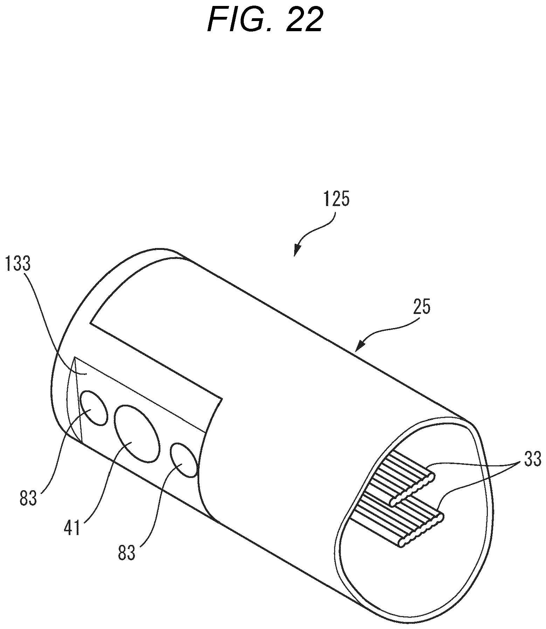

Fig. 22 is a perspective view of a rigid portion of the side-viewing endoscope in which the image-capturing module 47 for side viewing is accommodated. The lens barrel 51 of the image-capturing module 47 for side viewing is aligned with the window through hole 39 formed in a cutout side surface 133 of the rigid portion 25. A pair of light sources 83 are disposed on the cutout side surface 133 in a direction along the axis 71 with the window through hole 39 interposed therebetween.

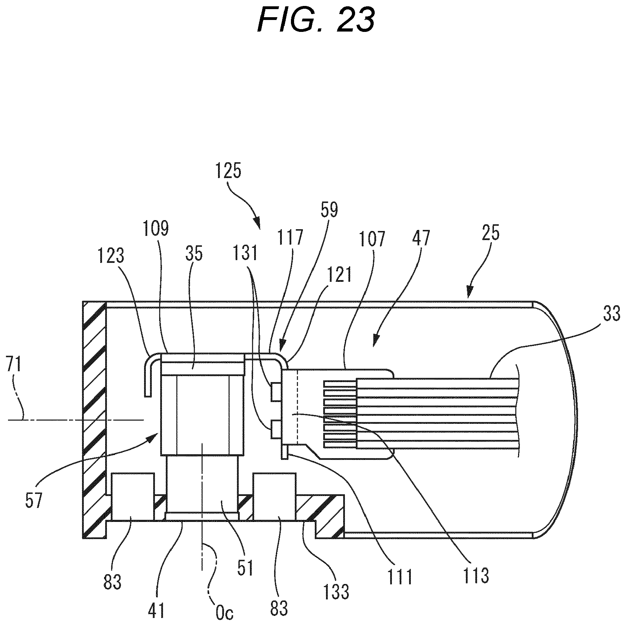

Fig. 23 is a cross-sectional view of the rigid portion of the side-viewing endoscope illustrated in Fig. 22. In the image-capturing module 47 for side viewing, the optical axis Oc of the optical system is orthogonal to the axis 71 of the rigid portion 25 (the lead-out direction of the transmission cable 33).

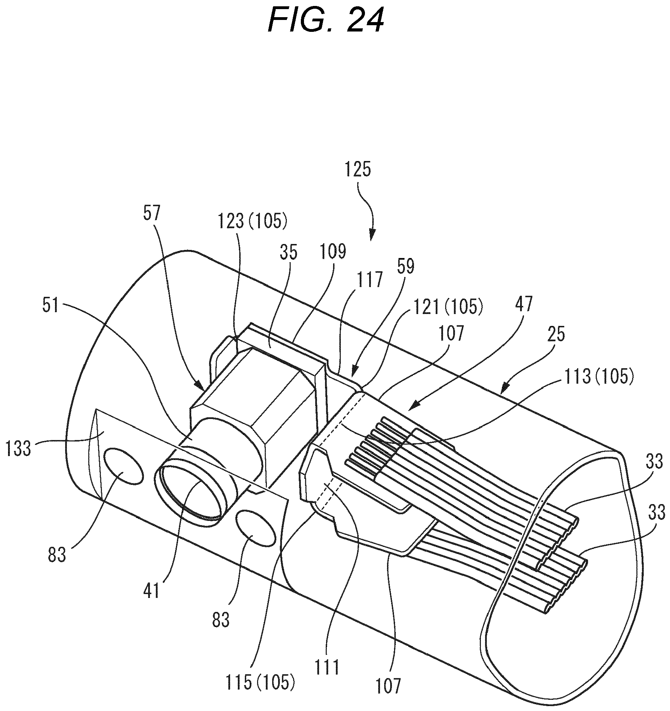

Fig. 24 is a perspective view in which the rigid portion of the side-viewing endoscope illustrated in Fig. 22 is seen through. In the image-capturing module 47 for side viewing, the optical axis Oc of the camera portion 57 is bent parallel to the both-wing connection portion 111 connected to the transmission cable 33. Therefore, the rigid portion 25 can be formed to have an inner diameter at which the entire length of the camera portion 57 is accommodated in a diameter direction thereof.

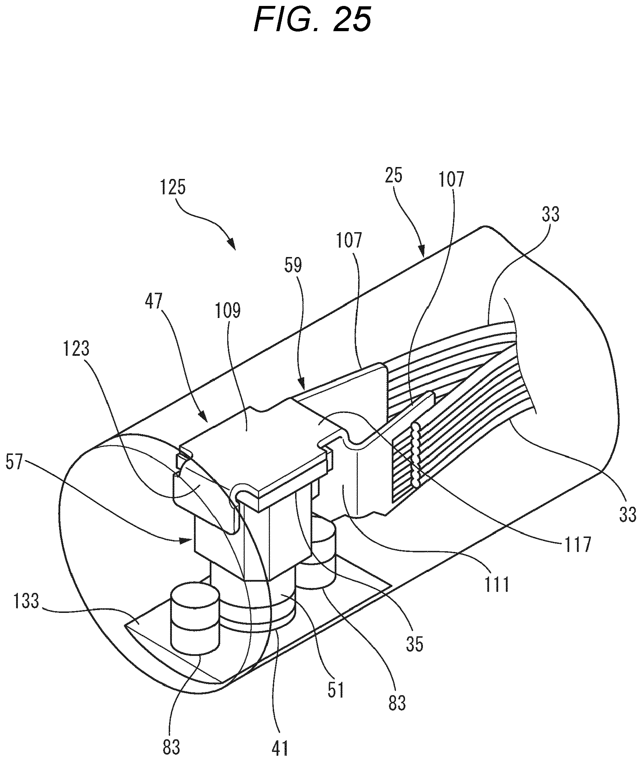

Fig. 25 is a perspective view of the rigid portion of the side-viewing endoscope illustrated in Fig. 24 as viewed from a side opposite to the optical system. The image-capturing module 47 for side viewing is bent from two parallel edge portions of the both-wing connection portion 111, and the transmission cable 33 is connected to each of the pair of both wing portions 107 that are parallel to each other. Accordingly, according to the image-capturing module 47 for side viewing using the flexible substrate 59, a large number of electric wires can be connected to the camera portion 57 at a high density by using a small accommodation space in the vicinity of the camera portion 57.

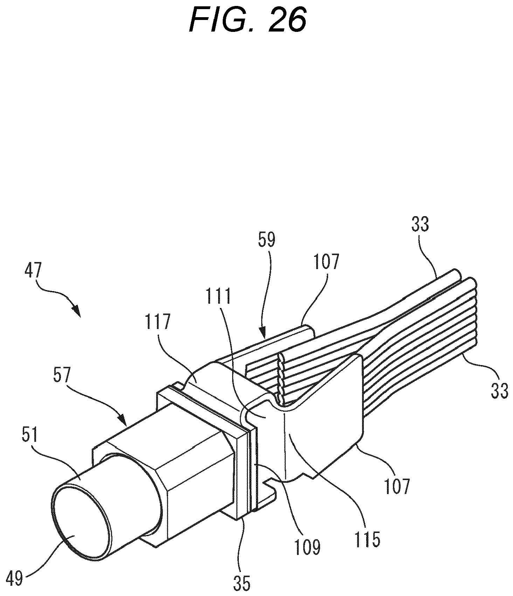

Fig. 26 is a perspective view of the image-capturing module 47 for direct viewing in which the bending portion 105 is in a bent state for a direct-viewing endoscope. In the image-capturing module 47 for direct viewing, in the T-shaped image-capturing module 47, the both wing portions 107 are bent in parallel at the first side-portion bending portion 113 and the second side-portion bending portion 115 with respect to the both-wing connection portion 111. In this case, the leg-like portion 109 is bent parallel to the both-wing connection portion 111 at the first neck-portion bending portion 119 and the second neck-portion bending portion 121.

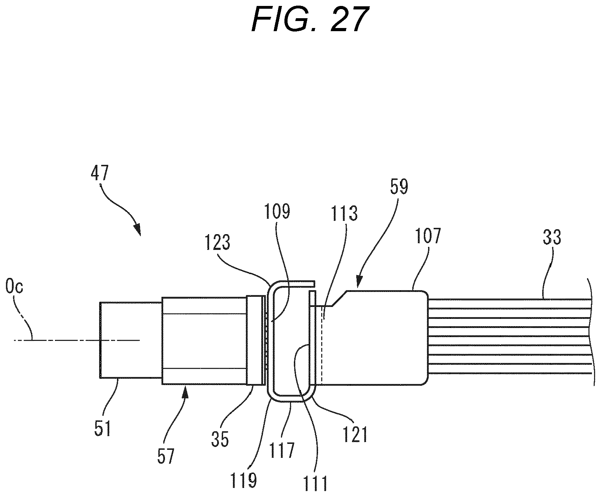

Fig. 27 is a side view of the image-capturing module 47 for direct viewing illustrated in Fig. 26. In the image-capturing module 47 for direct viewing, the both-wing connection portion 111 is bent in parallel with the image sensor 35. Accordingly, the image-capturing module 47 for direct viewing can be disposed such that the optical axis Oc of the camera portion 57 and the extending direction of the transmission cable 33 are in linear.

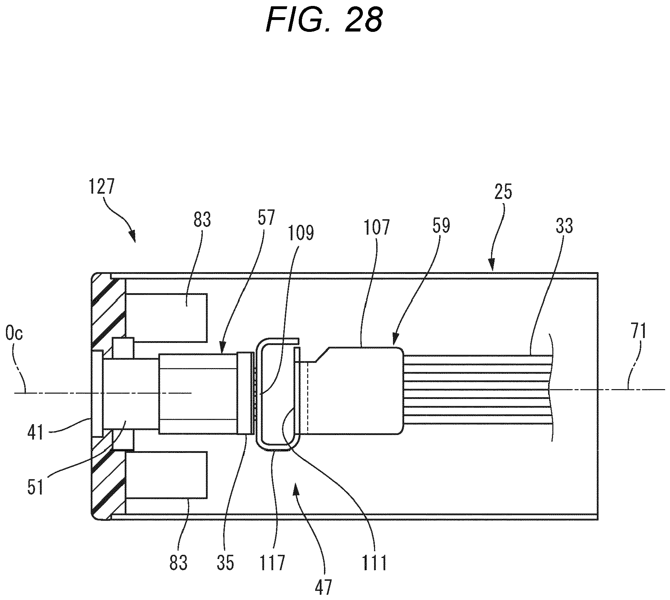

Fig. 28 is a cross-sectional view of a rigid portion of the direct-viewing endoscope in which the image-capturing module 47 for direct viewing is accommodated. In the image-capturing module 47 for direct viewing, the optical axis Oc of the optical system is oriented in the same direction as the axis 71 of the rigid portion 25 (the lead-out direction of the transmission cable 33).

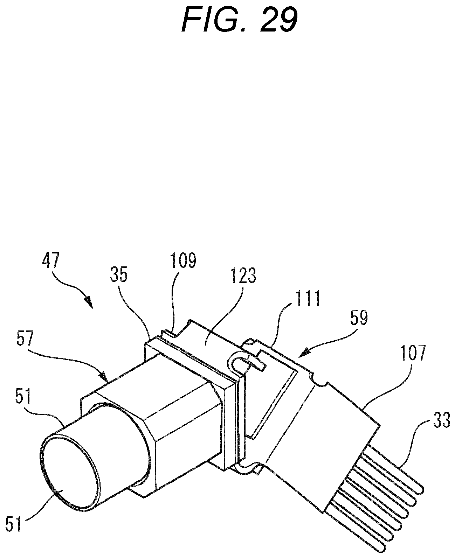

Fig. 29 is a perspective view of the image-capturing module 47 for oblique viewing in which the bending portion 105 is in a bent state for an oblique-viewing endoscope. In the image-capturing module 47 for oblique viewing, in the T-shaped image-capturing module 47, the both wing portions 107 are bent in parallel at the first side-portion bending portion 113 and the second side-portion bending portion 115 with respect to the both-wing connection portion 111. In this case, the both-wing connection portion 111 is bent at the second neck-portion bending portion 121 at an angle of about 45° with respect to the leg-like portion 109.

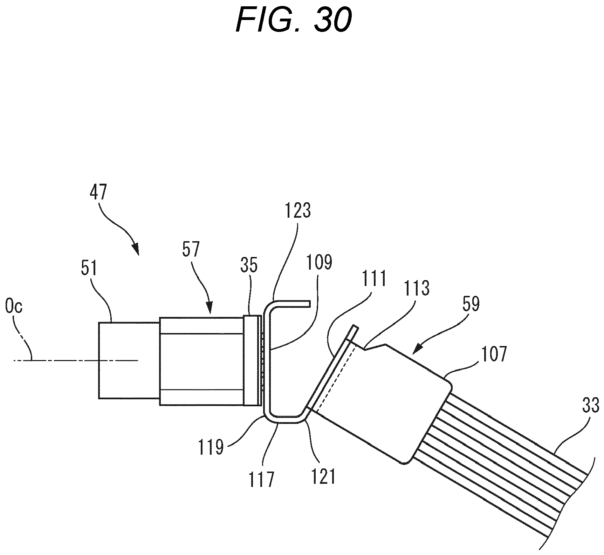

Fig. 30 is a side view of the image-capturing module 47 for oblique viewing illustrated in Fig. 29. In the image-capturing module 47 for oblique viewing, the both-wing connection portion 111 is bent so as to be at an intersection angle of about 45° with respect to the image sensor 35. Accordingly, the image-capturing module 47 for oblique viewing can be disposed such that the optical axis Oc of the camera portion 57 and the extending direction of the transmission cable 33 are inclined with respect to each other.

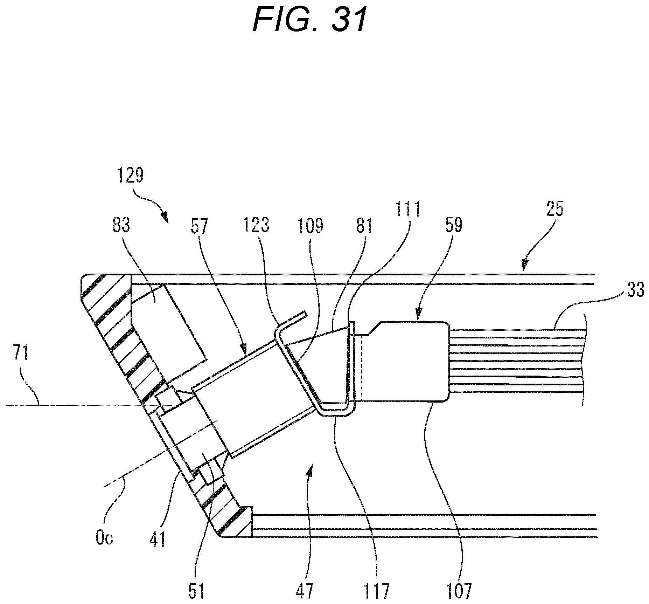

Fig. 31 is a cross-sectional view of a rigid portion of the oblique-viewing endoscope in which the image-capturing module 47 for oblique viewing is accommodated. The image-capturing module 47 for oblique viewing is used in an oblique-viewing endoscope in which the optical axis Oc of the optical system intersects the axis 71 of the rigid portion 25 (the lead-out direction of the transmission cable 33), and is incorporated in the oblique-viewing endoscope 129.

In the image-capturing module 47, the flexible substrate 59 is bent at the first neck-portion bending portion 119 and the second neck-portion bending portion 121, and thus the adhesive 81 (see Fig. 31) can be filled and fixed between the both-wing connection portion 111 and the leg-like portion 109 that face each other. In addition, it is needless to say that the image-capturing module 47 may not be filled with the adhesive 81 as long as the internal structure of the rigid portion 25 can hold the flexible substrate 59 for direct viewing, side viewing, and oblique viewing.

Although the cases where the endoscope is the direct-viewing endoscope 127, the side-viewing endoscope 125, and the oblique-viewing endoscope 129 are described in this application example, the same effects can be achieved by the direct viewing endoscope, the side viewing endoscope, and the oblique viewing endoscope 15 in each of which a pair of the endoscopes are incorporated.

In the present specification, the endoscope includes the monocular endoscope 103 and the compound-eye endoscope 101. The monocular endoscope 103 includes the direct-viewing endoscope 127, the side-viewing endoscope 125, and the oblique-viewing endoscope 129. The compound-eye endoscope 101 includes a direct viewing endoscope (not shown), a side viewing endoscope (not shown), and the oblique viewing endoscope 15.

### [Effects]

Next, effects of the oblique viewing endoscope 15 according to the first embodiment will be described.

The endoscope module (3D camera module 45) according to the first embodiment includes: two or more image-capturing modules 47 in each of which the lens barrel 51 housing an optical system, the image sensor 35, and the sensor holding member 53 that relatively fixes the lens barrel 51 and the image sensor 35 are assembled in the first atmosphere ENV1; and the sub-frame 63 that is assembled in the first atmosphere ENV1 and relatively fixes each of the two or more image-capturing modules 47. The sub-frame 63 and the two or more image-capturing modules 47 are accommodated and fixed in the outer shell 67 of the endoscope main body (that is, the main body of the oblique viewing endoscope 15) that is assembled in the second atmosphere ENV2 that is more clean than first atmosphere ENV1.

In the 3D camera module 45 according to the first embodiment, the two or more image-capturing modules 47 are accommodated and fixed in the outer shell of the endoscope main body. Each of the image-capturing modules 47 is assembled by the lens barrel 51 housing an optical system, the image sensor 35, and the sensor holding member 53 that relatively fixes the lens barrel 51 and the image sensor 35. In the image-capturing module 47, the optical system and the image sensor 35 are positioned relative to each other by the sensor holding member 53, so that image-capturing light from a subject optimally forms an image on a light receiving region of the image sensor 35. The image-capturing modules 47 assembled in this manner are assembled with the sub-frame 63 in the first atmosphere ENV1, so that the image-capturing modules 47 are relatively positioned and integrated.

The two or more image-capturing modules 47 integrated via the sub-frame 63 are accommodated in the outer shell of the endoscope main body assembled in the second atmosphere ENV2 that is more clean than the first atmosphere ENV1. That is, the endoscope accommodates and seals the two or more image-capturing modules 47, which are integrated, in the outer shell of the endoscope main body.

Here, the image-capturing module 47, in which the lens barrel 51 and the image sensor 35 are fixed using the sensor holding member 53, is assembled using a precise adjustment jig or the like. Further, the two or more image-capturing modules 47 are positioned relative to each other using a precise adjustment jig or the like and integrated. The assembling of a single image-capturing module 47, and the assembling of integrating two or more image-capturing modules 47 by the sub-frame 63, are performed in the same first atmosphere. Since each member assembled in the first atmosphere is not to be brought in contact with a patient, it is possible to perform the work in an atmosphere whose cleanliness is relatively relaxed.

On the other hand, the two or more image-capturing modules 47, which are assembled in the first atmosphere and integrated by the sub-frame 63, are accommodated in the outer shell in the second atmosphere. The second atmosphere is set to have higher cleanliness than the first atmosphere. The work of accommodating the two or more image-capturing modules 47, which are integrated, into the outer shell in the second atmosphere is performed in a clean process that receives official approval. That is, with respect to the 3D camera module 45, a process in which the cleanliness is particularly required can be completed by minimum work of merely accommodating the two or more image-capturing modules 47, which are integrated, into the outer shell.

As described above, for the 3D camera module 45, since the sub-frame 63 for relatively positioning and fixing the two or more image-capturing modules 47 is provided, the assembling can be performed using precise adjustment jigs or the like in the first atmosphere whose cleanliness is relatively relaxed. Accordingly, it is not necessary to install these precise adjustment jigs or the like in the second atmosphere having a high degree of cleanliness to perform the assembling.

As a result, for the 3D camera module 45, it is possible to separate an internal manufacturing process not including a patient contact portion from an external manufacturing process including a patient contact portion, and it is not necessary to maintain a particular cleanliness for the internal manufacturing process. Thus, it is possible to reduce the process management and the process construction cost. In particular, since a process of camera portion assembling requiring a precise adjustment jig or the like can be separated, it is possible to greatly reduce the process construction cost.

In addition, at least two of the two or more image-capturing modules 47 in the 3D camera module 45 captures images constituting a three-dimensional image.

In the 3D camera module 45, at least two of the two or more image-capturing modules 47 are image-capturing modules 47 for capturing images (for example, a left image and a right image) constituting a three-dimensional image. Specifications of the two image-capturing modules 47 for capturing images constituting a three-dimensional image match each other, and the optical axes of the two image-capturing modules 47 are parallel to each other. Accordingly, the optical axes of the two image-capturing modules 47 are separated from each other by a certain distance. Under such image-capturing conditions, in the 3D camera module 45, image-capturing surfaces of the two image-capturing modules 47 are set to be on the same plane. In the two image-capturing modules 47, a deviation between coordinates of images of the same point in the same subject, in two captured images respectively having coordinates, is the parallax px.

With the 3D camera module 45, it is possible to perform image processing on a plurality of different captured images using the parallax px acquired from a plurality of interlocking image-capturing modules 47 and display a stereoscopic image, in which depth information is reflected, on a display device.

In addition, in the 3D camera module 45, the sub-frame 63 has a pair of lens barrel insertion holes 65 into which the two lens barrels 51 are loosely fitted, and the outer peripheries of the two lens barrels 51 positioned with respect to each other are adhesively fixed to the inner peripheries of the lens barrel insertion holes 65.

In the 3D camera module 45, the sub-frame 63 has the pair of lens barrel insertion holes 65 into which the two lens barrels 51 are fitted (loosely fitted) with play. That is, a gap that allows movement exists between the lens barrel 51 and the lens barrel insertion hole 65.

Accordingly, the two lens barrels 51 can move in a state of being inserted through the lens barrel insertion holes 65, and rotation adjustment around an optical axis of the optical system and swing adjustment around an axis orthogonal to the optical axis are possible for the two lens barrels 51 in a state of being inserted through the lens barrel insertion holes 65. With respect to the two lens barrels 51, which are positioned with respect to each other and are positioned with respect to the sub-frame 63, and the sub-frame 63, the outer peripheries of the lens barrels 51 can be adhesively fixed by the adhesive 81 to the inner peripheries of the lens barrel insertion holes 65. The adhesive 81 is filled in a space around a side surface of the lens barrel 51 extending from an opening of the lens barrel insertion hole 65 to the sensor holding member 53, and is caused to cure, so that the adhesive strength and the positional accuracy can be maintained over a long period of time.

As a result, the two lens barrels 51 are positioned with respect to the sub-frame 63. Therefore, in the 3D camera module 45, when the sub-frame 63 is positioned at a predetermined position with respect to the outer shell 67, the optical systems of the two image-capturing modules 47 are simultaneously positioned with respect to the outer shell 67 (rigid portion 25).

In addition, the oblique viewing endoscope 15 according to the first embodiment includes: two or more image-capturing modules 47 in each of which the lens barrel 51 housing an optical system, the image sensor 35, and the sensor holding member 53 that relatively fixes the lens barrel 51 and the image sensor 35 are assembled in the first atmosphere ENV1; the sub-frame 63 that is assembled in the first atmosphere ENV1 and relatively fixes each of the two or more image-capturing modules 47; and the outer shell portion (for example, the outer shell 67) that accommodates and fixes the sub-frame 63 and the two or more image-capturing modules 47 and is assembled in the second atmosphere ENV2 that is more clean than the first atmosphere ENV1. The outer shell 67 includes the endoscope tip-end component 37 to which the sub-frame 63 is fixed, and the sheath 43 in which the tubular tip end opening 69 is closed by the endoscope tip-end component 37.

In the endoscope according to the first embodiment, the outer shell 67 of the endoscope main body includes the endoscope tip-end component 37 and the sheath 43. The endoscope tip-end component 37 is formed in a disk shape or an elliptical plate shape. The endoscope tip-end component 37 is made of, for example, a metal such as stainless steel. An outer circumferential surface of the endoscope tip-end component 37 in a thickness direction is adhesively fixed to an inner circumferential surface of the sheath 43. In the endoscope tip-end component 37 whose outer circumferential surface is fixed to the sheath 43, a projecting portion having an outer diameter larger than that of the outer circumferential surface by a thickness of the sheath 43 is formed at a tip end thereof. Therefore, the projecting portion having the outer diameter, of the endoscope tip-end component 37, flush with the sheath 43 having an outer diameter.

Accordingly, in the endoscope, the outer circumferential surface of the endoscope tip-end component 37 is inserted into the tip end opening 69 of the sheath 43, and a tip end of the sheath 43 abuts the projecting portion so as to be adhesively fixed, whereby the outer shell 67 having the same outer diameter without a step and having high strength can be assembled by simple work.

In addition, the plug portion 21 enabling transmission and reception of electric power and various signals to and from the image-capturing module 47 via the transmission cable 33 inserted through the sheath 43 is connected to the rear end portion of the sheath 43.

Accordingly, data of a captured image captured by the oblique viewing endoscope 15 can be transmitted to the video processor 13, and a highly accurate image captured by the oblique viewing endoscope 15 can be displayed on the monitor 17 at the time of surgery, examination, or the like performed by a doctor or the like.

Further, in the endoscope, the endoscope tip-end component 37 and the tip end portion of the sheath 43 adhesively fixed to the endoscope tip-end component 37 constitute the cylindrical rigid portion 25. The tip end opening 69 of the sheath 43 is opened to be inclined with respect to the virtual plane 73 perpendicular to the axis 71 of the rigid portion 25, the endoscope tip-end component 37 is inclined with respect to the virtual plane 73 to close the tip end opening 69, and the chamfered portion 79 is formed at the forward-inclined tip end portion 77 on the inclined tip end surface 75 of the endoscope tip-end component 37. When the endoscope is a direct-viewing endoscope, the tip end opening 69 of the sheath 43 is opened without being inclined with respect to the virtual plane 73 perpendicular to the axis 71 of the rigid portion 25. The endoscope tip-end component 37 may close the tip end opening 69 of the sheath 43, and the chamfered portion 79 (for example, see the chamfered portion 79) may be formed on a tip end surface of the endoscope tip-end component 37.

In this endoscope, the tip end opening 69 of the sheath 43 is opened to be inclined with respect to the virtual plane 73 perpendicular to the axis 71 of the rigid portion 25. The endoscope tip-end component 37 is inclined with respect to the virtual plane 73 to close the tip end opening 69. That is, the endoscope is the oblique viewing endoscope 15 in which the endoscope tip-end component 37 is inclined with respect to the virtual plane 73 perpendicular to the axis 71 of the rigid portion 25.

In the endoscope, on the inclined tip end surface 75 of the inclined endoscope tip-end component 37, the chamfered portion 79 is formed at the forward-inclined tip end portion 77 that is the most tip end. In the cylindrical rigid portion 25, the forward-inclined tip end portion 77 becomes sharp due to the inclination of the endoscope tip-end component 37. By chamfering the forward-inclined tip end portion 77 that becomes sharp, for example, it is possible to prevent damage to a tube wall at the time of insertion of a body cavity such as a blood vessel, thereby improving safety. Even if the endoscope is a direct-viewing endoscope, the improvement in safety can be achieved similarly by forming the chamfered portion 79 (for example, see the chamfered portion 79) on the tip end surface of the endoscope tip-end component 37.

Further, in an endoscope, a plurality of window through holes 39 are provided in the endoscope tip-end component 37, and each of the window through holes 39 is hermetically sealed by the cover glass 41.

In this endoscope, the plurality of window through holes 39 are provided in the endoscope tip-end component 37. The window through hole 39 is sealed with the cover glass 41. Therefore, when the endoscope tip-end component 37 is attached to close the tip end opening 69 of the sheath 43, the inside of the endoscope is hermetically shielded from the outside by the outer shell 67 that is composed of the endoscope tip-end component 37 and the sheath 43.

Through each of the cover glasses 41 provided to close the plurality of window through holes 39, image-capturing light can be taken in from the outside and illumination light can be emitted from the inside. Accordingly, in the endoscope, the tip end opening 69 of the sheath 43 is sealed by the endoscope tip-end component 37 that enables incidence of image-capturing light and emission of illumination light, and thus the internal structure can be sealed at the same time while securing a light receiving function and an illumination function.

Further, in an endoscope, the endoscope tip-end component 37 has the sub-frame fixing recess 87 for relatively fixing the sub-frame 63.

In this endoscope, the endoscope tip-end component 37 has the sub-frame fixing recess 87 for relatively fixing the sub-frame 63. When the image-capturing module 47 is fixed to the sub-frame fixing recess 87, the two image-capturing modules 47 are positioned with respect to each other via the sub-frame 63, and are also positioned with respect to the endoscope tip-end component 37 via the sub-frame 63.

Accordingly, the two image-capturing modules 47 are positioned and fixed to the endoscope tip-end component 37 via the sub-frame 63 and the sub-frame fixing recess 87, and at the same time, can be positioned with respect to the respective window through holes 39 closed by the cover glasses 41.

In addition, in an endoscope, the light source 83 that emits illumination light to the outside of the outer shell 67 is disposed on the back surface of at least one cover glass 41.

In this endoscope, the light source 83 that emits illumination light to the outside of the outer shell 67 is disposed on the back surface of at least one cover glass 41. The light source 83 may be, for example, an LED adhesively fixed to the back surface of the endoscope tip-end component 37.

In the endoscope in which the light source 83 is directly attached to the endoscope tip-end component 37, a sufficient amount of light can be obtained and the subject can be illuminated sufficiently brightly, as compared with a case of a light guide in which a radius of curvature is reduced and a radiation loss is increased.

In the endoscope, the light source 83 may be a light emission diode (LED), and the LED and a cooling mechanism of the LED may be disposed as the light source 83 on the back surface of the cover glass 41.

Accordingly, when the LED is used as the light source 83, a measure for radiating heat generated from the LED is taken, and an adverse effect produced due to heat propagation to the endoscope tip-end component 37 can be suppressed.

In the endoscope, the light source 83 may be an optical fiber that is covered with the sheath 43 and extends from a base end side of the endoscope to the endoscope tip-end component 37.

Accordingly, since the use of the LED at the tip end can be avoided, it is easy to implement size reduction of the endoscope tip-end component 37 as compared with the case where the LED is used as the light source, and since light can be guided from the base end side of the endoscope to the endoscope tip-end component 37 by the optical fiber, it is possible to sufficiently brightly illuminate the subject as in the case of the LED.

In addition, the oblique viewing endoscope 15 according to the first embodiment includes: two or more image-capturing modules 47 each of which includes the image sensor 35, the lens barrel 51 housing an optical system, and the sensor holding member 53 that relatively fixes the lens barrel 51 and the image sensor 35; the sub-frame 63 that relatively fixes each of the two or more image-capturing modules 47; and the outer shell portion (for example, the outer shell 67) that accommodates the sub-frame 63 and the two or more image-capturing modules 47.

In the endoscope structure according to the first embodiment, the two or more image-capturing modules 47 are accommodated in the outer shell of the endoscope main body. Each of the image-capturing modules 47 is assembled by the lens barrel 51 housing an optical system, the image sensor 35, and the sensor holding member 53 that relatively fixes the lens barrel 51 and the image sensor 35. In the image-capturing module 47, the optical system and the image sensor 35 are positioned relative to each other by the sensor holding member 53, so that image-capturing light from a subject optimally forms an image on a light receiving region of the image sensor 35. The image-capturing modules 47 assembled in this manner are relatively positioned and integrated by the sub-frame 63.

The two or more image-capturing modules 47 integrated via the sub-frame 63 are accommodated in the outer shell of the endoscope main body. That is, in the endoscope structure, the two or more image-capturing modules 47 that are integrated can be accommodated and sealed in the outer shell of the endoscope main body.

In the oblique viewing endoscope 15, the sub-frame 63 is isolated from the outside of the outer shell 67.

In this endoscope structure, the sub-frame 63 is isolated from the outside of the outer shell 67. The image-capturing module 47, in which the lens barrel 51 and the image sensor 35 are fixed using the sensor holding member 53, is assembled using a precise adjustment jig or the like. Further, the two or more image-capturing modules 47 are positioned relative to each other using a precise adjustment jig or the like and integrated. The assembling of a single image-capturing module 47, and the assembling of integrating two or more image-capturing modules 47 by the sub-frame 63, are assembling of a portion that is not to be in contact with a patient. Therefore, it is possible to perform work in an atmosphere whose cleanliness is relatively relaxed.

On the other hand, the work of accommodating the two or more image-capturing modules 47, which are integrated, into the outer shell is assembling of a portion that is to come into contact with the patient, and thus needs to be performed in a clean process that receives official approval.

Therefore, in this endoscope structure, a process in which cleanliness is particularly required can be completed by minimum work of simply accommodating the two or more image-capturing modules 47, which are integrated, into an outer shell.

As described above, for the oblique viewing endoscope 15, since the sub-frame 63 for relatively positioning and fixing the two or more image-capturing modules 47 is provided, the assembling can be performed using precise adjustment jigs or the like in a manufacturing environment whose cleanliness is relatively relaxed. Accordingly, it is not necessary to install these precise adjustment jigs or the like in a manufacturing environment having a high degree of cleanliness to perform the assembling.

As a result, for the oblique viewing endoscope 15, it is possible to separate an internal manufacturing process not including a patient contact portion from an external manufacturing process including a patient contact portion, and it is not necessary to maintain a particular cleanliness for the internal manufacturing process. Thus, it is possible to reduce the process management and the process construction cost. In particular, since a process of camera portion assembling requiring a precise adjustment jig or the like can be separated, it is possible to greatly reduce the process construction cost.

A method of manufacturing the endoscope according to the first embodiment includes, to be performed in a first atmosphere, a step of assembling the image-capturing module 47 by relatively fixing the lens barrel 51 housing an optical system and the image sensor 35 by using the sensor holding member 53, and a step of relatively fixing each of two or more image-capturing modules 47 by using the sub-frame 63, and includes, to be performed in a second atmosphere different from the first atmosphere, a step of fixing the sub-frame 63 to the back surface of the endoscope tip-end component 37, and a step of forming the outer shell 67 of the endoscope main body by fixing the endoscope tip-end component 37 to the tip end opening 69 of the tubular sheath 43, and at the same time, hermetically sealing the sub-frame 63 and the two or more image-capturing modules 47 in the outer shell.

In the endoscope manufacturing method according to the first embodiment, the oblique viewing endoscope 15 is assembled in two different atmospheres of first atmosphere ENV1 and second atmosphere ENV2. The second atmosphere ENV2 is set to have a higher cleanliness than the first atmosphere ENV1.

In the first atmosphere, the image-capturing module 47 is assembled by fixing the lens barrel 51 and the image sensor 35 by using the sensor holding member 53. The image-capturing module 47 is assembled using a precise adjustment jig or the like. Further, in the first atmosphere, the two or more image-capturing modules 47 are positioned relative to each other by using a precise adjustment jig or the like, and are integrally assembled via the sub-frame 63.

That is, the assembling of a single image-capturing module 47, and the assembling of integrating two or more image-capturing modules 47 by the sub-frame 63, are performed in the same first atmosphere. Since each member assembled in the first atmosphere is not to be brought in contact with a patient, it is possible to perform the work in an atmosphere whose cleanliness is relatively relaxed.

On the other hand, the two or more image-capturing modules 47, which are assembled in the first atmosphere and integrated by the sub-frame 63, are accommodated into the outer shell in the second atmosphere ENV2. That is, with respect to the 3D camera module 45, a process in which the cleanliness is particularly required is completed by minimum work of merely accommodating the two or more image-capturing modules 47, which are integrated, into the outer shell.

As described above, with the endoscope manufacturing method, since the sub-frame 63 for relatively positioning and fixing the two or more image-capturing modules 47 is used, the assembling can be performed using precise adjustment jigs or the like in the first atmosphere whose cleanliness is relatively relaxed. Accordingly, it is not necessary to install these precise adjustment jigs or the like in the second atmosphere having a high degree of cleanliness to perform the assembling.

As a result, with the endoscope manufacturing method, it is possible to separate an internal manufacturing process not including a patient contact portion from an external manufacturing process including a patient contact portion, and it is not necessary to maintain a particular cleanliness for the internal manufacturing process. Thus, it is possible to reduce the process management and the process construction cost. In particular, since a process of camera portion assembling requiring a precise adjustment jig or the like can be separated, it is possible to greatly reduce the process construction cost.

Therefore, according to the 3D camera module 45, the oblique viewing endoscope 15, the endoscope structure, and the endoscope manufacturing method according to the first embodiment, the assembling process requiring cleanliness and the assembling process not requiring cleanliness can be separated, and an increase in manufacturing cost can be suppressed.

The compound-eye endoscope 101 according to the first embodiment includes: the two or more image-capturing modules 47 that are formed in the same outer shape and each of which includes the lens barrel 51 housing an optical system (for example, the lens 49), the image sensor 35, and the sensor holding member 53 that relatively fixes the lens barrel 51 and the image sensor 35; the sub-frame 63 that relatively fixes each of the two or more image-capturing modules 47; and the outer shell 67 that accommodates and fixes the sub-frame 63 and the two or more image-capturing modules 47. Each of the two or more image-capturing modules 47 has a performance evaluation characteristic satisfying a predetermined correlation with each other as a result of a performance evaluation test of a predetermined technical item including 3D image-capturing performance measured in advance.

In the compound-eye endoscope 101 according to the first embodiment, the lens barrel 51 and the image sensor 35 are fixed by the sensor holding member 53 to form one of the image-capturing modules 47 having the same outer shape. Even a single image-capturing module 47 is able to operate. That is, the compound-eye endoscope 101 can be assembled by combining two identical image-capturing modules 47, and the monocular endoscope 103 can be assembled by using a single image-capturing module 47. In the compound-eye endoscope 101, two or more image-capturing modules 47 are relatively fixed by the sub-frame 63. The two or more image-capturing modules 47 integrated by the sub-frame 63 are fixed and accommodated inside the outer shell 67.

The image-capturing module 47 accommodated in the outer shell has a performance evaluation characteristic satisfying a predetermined correlation with each other as a result of a technical performance evaluation test including 3D image-capturing performance. In this case, each of the image-capturing modules 47 may have commonality under a predetermined correlation even in technical evaluation characteristics starting with the outer shape. That is, component sharing is possible. Accordingly, the image-capturing module 47 can be shared by the monocular endoscope and the compound-eye endoscope 101. That is, the image-capturing module 47 as a component can be shared in other endoscopes (monocular endoscopes). As a result, it is possible to increase the production number of single image-capturing module, and it is possible to reduce the component cost thanks to the mass production effect. Further, the quality can be stabilized by making a large amount of the same components.

In addition, in the compound-eye endoscope 101, since the two or more image-capturing modules 47 are fixed by the sub-frame 63 having a simple plate shape, the structure can be simplified and further the component cost can be reduced as compared with a case of, for example, a box-shaped mounting member used in the electronic endoscope apparatus disclosed in Patent Literature 1. Since a screw or the like, which is necessary for a mounting member in the related art, is not necessary, the number of all components to be produced can be reduced, and both the component cost and the management cost can be reduced. In addition, the number of assembling processes, production jigs, and tools can be reduced with the commonality of the components described above, and the assembling cost can also be reduced.

In addition, in the compound-eye endoscope 101, since the two image-capturing modules 47 are fixed by the sub-frame 63 having a simple plate shape and the imaging modules 47 basically have commonality, even if one of the image-capturing modules 47 is in a bad condition during assembly, the image-capturing module 47 can be replaced immediately, and the recovery is easy. In other words, even when one of the image-capturing modules 47 fails due to an electrical trouble (disconnection, electrostatic breakdown, or the like) during assembly, it is not necessary to discard the all two modules.

As described above, since the compound-eye endoscope 101 is configured to include two or more image-capturing modules 47 having a predetermined correlation in the technical evaluation characteristics, for example, it is possible to reduce the manufacturing cost while increasing the number of non-defective products in which a good 3D vision is obtained.

In the compound-eye endoscope 101, unlike the electronic endoscope apparatus described in the background art in which it is necessary to intentionally form an eccentric optical system for 3D adjustment (adjustment of the relative direction of both eyes), there is no cost for manufacturing an eccentric optical system, and accordingly there is no occurrence of decrease in image quality.

In the compound-eye endoscope 101, the predetermined correlation is a relationship in which variation in values indicated by the performance evaluation characteristics of the respective image-capturing modules 47 is suppressed (that is, a relationship within a predetermined range).

In this compound-eye endoscope 101, for example, the two image-capturing modules 47 have a predetermined correlation. The predetermined correlation is a relationship in which variation in a characteristic evaluation value is suppressed. An example of the characteristic evaluation value is a best focus position. The image-capturing module 47 assembled with a tolerance can be divided into a non-standard NG product beyond the tolerance and a standard non-defective product within the tolerance. The standard non-defective product further includes a plurality of solids (image-capturing module 47) whose characteristic evaluation value (for example, the best focus position) varies. The best focus position is related to a visual observation range of a person.

At the time of observation, when there is a large difference (large difference in variation) between best focus positions of the image-capturing modules 47 used for the left eye and the right eye in the compound-eye endoscope 101, it is easy for the user to feel fatigue. For example, in the case of the combination of the product of difference (rank) C2 and the product of rank A1 shown in Fig. 8, a feeling of discomfort in the 3D vision is caused and a feeling of fatigue is increased.

Therefore, in the compound-eye endoscope 101, two image-capturing modules 47 having a predetermined correlation in which the variation in the characteristic evaluation value is suppressed are incorporated. For example, two products of the rank C2 shown in Fig. 8 are combined, and they are incorporated into the sub-frame 63. Further, for example, the products of the rank C2 and rank D2 or the products of the rank C2 and B2 are combined, and they are incorporated into the sub-frame 63. Accordingly, it is possible to obtain the compound-eye endoscope 101 with which the user is less likely to feel fatigue even when using the compound-eye endoscope 101 for a long time.

In this case, depending on the combination, the image-capturing modules 47 that are inappropriate can be used as non-defective products by combining at the same time the image-capturing modules 47 that have a correlation of complementing each other. That is, the yield of the image-capturing module 47 is improved. This also makes it possible to reduce the manufacturing cost of the compound-eye endoscope 101 and the monocular endoscope 103.

In the compound-eye endoscope 101, a predetermined correlation is a relationship in which the performance evaluation characteristics of the respective image-capturing modules 47 are different from each other.

In this compound-eye endoscope 101, for example, the two image-capturing modules 47 have a predetermined correlation. The predetermined correlation is a mutual relationship in different technical evaluation characteristics. Examples of the different technical evaluation characteristics include a focal depth and resolution. In addition, as the different technical evaluation characteristic, for example, only the focal depth (actual focal position) can be cited. As a characteristic performance improving method of a 3D image observation apparatus, there is one in which specific evaluation characteristics (that is, focal positions) of two image-capturing modules 47 are intentionally made different on the left and right. With the compound-eye endoscope 101 configured by using the two image-capturing modules 47 having such a correlation, since the left and right sides of a synthesized image in the brain complement each other, it is possible to obtain a wide recognition acceptance range.

In addition, the endoscope (for example, the side-viewing endoscope 125, the direct-viewing endoscope 127, and the oblique-viewing endoscope 129) according to the first embodiment includes the image-capturing module 47. The image-capturing module 47 includes the lens barrel 51 housing an optical system (for example, the lens 49), the image sensor 35, the sensor holding member 53 that relatively fixes the lens barrel 51 and the image sensor 35, and the flexible substrate 59 that transmits a signal of the image sensor 35 to the transmission cable 33. The flexible substrate 59 can be bent at an arbitrary number of bending portions 105. Here, the arbitrary number is not limited to an integer of 2 or more, and includes 1. The flexible substrate 59 having only one bending portion 105 corresponds to a case where the flexible substrate 59 has an L shape.

In the endoscope according to the first embodiment, the flexible substrate 59 that connects the image sensor 35 and the transmission cable 33 is appropriately bent at the first side-portion bending portion 113, the second side-portion bending portion 115, the first neck-portion bending portion 119, the second neck-portion bending portion 121, and the tip-end bending portion 123, so that it is possible to variously change the direction of the optical axis Oc of the optical system and the lead-out direction of the transmission cable 33.

Accordingly, unlike the electronic endoscope apparatus disclosed in Patent Literature 1, it is not necessary to manufacture a dedicated CCD module for manufacturing the oblique-viewing endoscope 129, the side-viewing endoscope 125, and the direct-viewing endoscope 127. That is, the bending portion 105 of the flexible substrate 59 developed in the T shape is variously bent, so that one type of image-capturing module 47 can be applied to the oblique-viewing endoscope 129, the side-viewing endoscope 125, and the direct-viewing endoscope 127. Therefore, the image-capturing module 47 is improved in versatility, is suitable for mass production, and can be manufactured at low cost. As a result, the manufacturing cost of the endoscope can be reduced.

In an endoscope, when the image-capturing module 47 is incorporated into the rigid portion 25 having rigidity provided at the tip end of the flexible portion 23, the bending portion 105 is in a bent state according to the shape of the rigid portion 25.

With respect to this endoscope, the image-capturing module 47 can be supplied to the user with the flexible substrate 59 in a developed state. On the other hand, the user can incorporate the image-capturing module 47 by bending the image-capturing module 47 in accordance with each of the oblique-viewing endoscope 129, the side-viewing endoscope 125, and the direct-viewing endoscope 127, and it is not necessary to hold a plurality of types of image-capturing modules 47 as a stock for each type of endoscope. That is, only one type of image-capturing module 47 may be held in stock. The manufacturer of the image-capturing module 47 only needs to manufacture one type of image-capturing module 47 to be applied to the oblique-viewing endoscope 129, the side-viewing endoscope 125, and the direct-viewing endoscope 127, and thus it is possible to increase the production number of the image-capturing module 47 and to reduce the component cost thanks to the mass production effect. In addition, since the number of product types can be reduced, the management cost can be reduced.

In addition, in an endoscope, in the image-capturing module 47, the optical axis Oc of the optical system is oriented in the same direction as the axis 71 of the rigid portion 25, and the bending portion 105 is in a bent state.

In this endoscope (for example, the direct-viewing endoscope 127), in the T-shaped image-capturing module 47, the both wing portions 107 are bent in parallel at the first side-portion bending portion 113 and the second side-portion bending portion 115 with respect to the both-wing connection portion 111. In the T-shaped image-capturing module 47, the leg-like portion 109 is bent in parallel with the both-wing connection portion 111 at the first side-portion bending portion 113 and the second neck-portion bending portion 121, so that the image-capturing module 47 is used for a direct-viewing endoscope in which the optical axis Oc of the optical system is oriented in the same direction as the axis 71 of the rigid portion 25 (the lead-out direction of the transmission cable 33).

In addition, in an endoscope, in the image-capturing module 47, the bending portion 105 is in a bent state such that the optical axis Oc of the optical system is orthogonal to the axis 71 of the rigid portion 25.

In this endoscope (for example, the side-viewing endoscope 125), in the T-shaped image-capturing module 47, the both wing portions 107 are bent in parallel at the first side-portion bending portion 113 and the second side-portion bending portion 115 with respect to the both-wing connection portion 111. In the T-shaped image-capturing module 47, the leg-like portion 109 is bent at the second neck-portion bending portion 121 to be perpendicular to the both-wing connection portion 111, so that the image-capturing module 47 is used for a side-viewing endoscope in which the optical axis Oc of the optical system is orthogonal to the axis 71 of the rigid portion 25 (the lead-out direction of the transmission cable 33).

In addition, in an endoscope, in the image-capturing module 47, the bending portion 105 is in a bent state such that the optical axis Oc of the optical system intersects the axis 71 of the rigid portion 25.

In this endoscope (for example, the oblique-viewing endoscope 129), in the T-shaped image-capturing module 47, the both wing portions 107 are bent in parallel at the first side-portion bending portion 113 and the second side-portion bending portion 115 with respect to the both-wing connection portion 111. In the T-shaped image-capturing module 47, the leg-like portion 109 is bent at the second neck-portion bending portion 121 to be at about 45° with respect to the both-wing connection portion 111, so that the image-capturing module 47 is used for an oblique-viewing endoscope in which the optical axis Oc of the optical system intersects the axis 71 of the rigid portion 25 (the lead-out direction of the transmission cable 33).

Therefore, the compound-eye endoscope 101 according to the first embodiment is excellent in mass productivity and can reduced the manufacturing cost. Further, the endoscope (for example, the side-viewing endoscope 125, the direct-viewing endoscope 127, and the oblique-viewing endoscope 129) according to the first embodiment is high in versatility and excellent in mass productivity, and can reduce the manufacturing cost.

Although various embodiments have been described above with reference to the drawings, it is needless to say that the present disclosure is not limited to such examples. It will be apparent to those skilled in the art that various alterations, modifications, substitutions, additions, deletions, and equivalents can be conceived within the scope of the claims, and it should be understood that such changes also belong to the technical scope of the present disclosure. Components in the various embodiments described above may be combined freely within a range not deviating from the spirit of the invention.

### INDUSTRIAL APPLICABILITY

The present disclosure is useful as a compound-eye endoscope that is excellent in mass productivity and can reduce the manufacturing cost.

## Claims

1. A compound-eye endoscope, comprising:
two or more image-capturing modules, each of the two or more image-capturing modules including a lens barrel housing an optical system, an image sensor, and a sensor holding member that relatively fixes the lens barrel and the image sensor;
a sub-frame that relatively fixes each of the two or more image-capturing modules; and
an outer shell that accommodates and fixes the sub-frame and the two or more image-capturing modules.

2. The compound-eye endoscope according to claim 1,
wherein each of the two or more image-capturing modules has a performance evaluation characteristic satisfying a predetermined correlation with each other as a result of a performance evaluation test of a predetermined technical item including image-capturing performance measured in advance.

3. The compound-eye endoscope according to claim 2,
wherein the predetermined correlation is a relationship in which variation in a value indicated by the performance evaluation characteristic of each of the image-capturing modules is within a predetermined range.

4. The compound-eye endoscope according to claim 2,
wherein the predetermined correlation is a relationship in which the performance evaluation characteristics of the respective image-capturing modules are different from each other.
